(19) Europäisches Patentamt
European Patent Office
Office européen des brevets

(11) Publication number: **0 358 758 A1**

# (12) EUROPEAN PATENT APPLICATION published in accordance with Art. 158(3) EPC

(21) Application number: **88903364.3**

(22) Date of filing: **08.04.88**

(86) International application number: **PCT/JP88/00359**

(87) International publication number: **WO 88/07892 (20.10.88 88/23)**

(51) Int. Cl.[5]: **B01J 20/22 , B01J 20/26 , A61M 1/36 , A61K 35/14 , G01N 33/50**

(30) Priority: **09.04.87 JP 87640/87**
**14.07.87 JP 173893/87**

(43) Date of publication of application: **21.03.90 Bulletin 90/12**

(84) Designated Contracting States: **BE CH DE FR GB LI SE**

(71) Applicant: **Terumo Kabushiki Kaisha**
**No. 44-1, Hatagaya 2-chome Shibuya-ku**
**Tokyo 151(JP)**

(72) Inventor: **YURA, Yoshifumi**
**Terumo Kabushiki Kaisha 2656-1, Ohbuchi**
**Fuji-shi Shizuoka 417(JP)**
Inventor: **YAMAMOTO, Yuichi**
**Terumo Kabushiki Kaisha 2656-1, Ohbuchi**
**Fuji-shi Shizuoka 417(JP)**
Inventor: **SAMEJIMA, Tadashi**
**Terumo Kabushiki Kaisha 2656-1, Ohbuchi**
**Fuji-shi Shizuoka 417(JP)**
Inventor: **AKAIKE, Toshihiro**
**15-23, Shimohoya 4-chome**
**Hoya-shi Tokyo 202(JP)**

(74) Representative: **Gillard, Marie-Louise et al**
**Cabinet Beau de Loménie 55, Rue d'Amsterdam**
**F-75008 Paris(FR)**

## (54) B LYMPHOCYTE SEPARATING MATERIAL AND BODY FLUID CLARIFYING MATERIAL.

(57) A B lymphocyte separating material, a method for separating B lymphocytes using same, and a separator are disclosed. The separating material comprises a water-insoluble solid substance having immobilized, directly or via a spacer, on the surface thereof an organically synthesized ligand having high affinity for immunoglobulin. A body fluid clarifying material and a body fluid clarifying apparatus using same are also disclosed. The material comprises a water-insoluble carrier having immobilized, directly or via a spacer, on the surface thereof a dipeptide or its derivative.



Xerox Copy Centre

FIG. 1

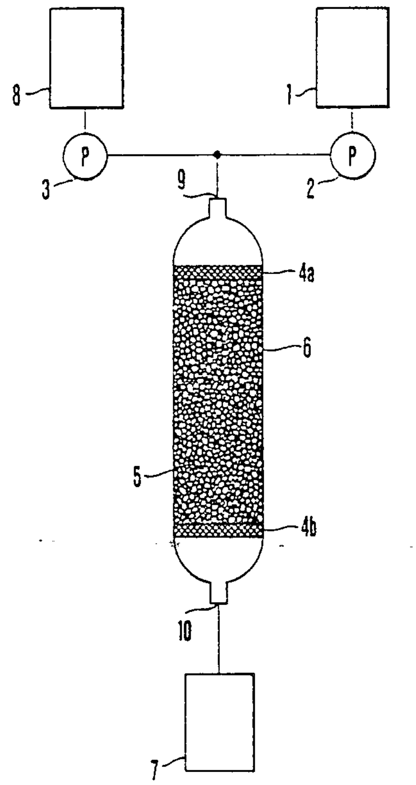

SPECIFICATION

# B LYMPHOCYTE SEPARATING MATERIAL AND BODY FLUID PURIFYING MATERIAL

[Technical Field]

This invention relates to a B lymphocyte separating material and a body fluid purifying material.

This invention also relates to a B lymphocyte separating material, a method for the separation of B lymphocyte, and a separating device. More particularly, this invention discloses a B lymphocyte separating material, a separating method, and a separating device for expediently and inexpensively in high yield with high purity the B lymphocytes from such a B lymphocyte-containing liquid as a lymphocyte fraction-floating cell suspension or a leukocyte fraction obtained from the blood or the vital tissues while minimizing a functional damage on the cell component in the liquid under treatment.

This invention further relates to a body fluid purifying material, a body fluid purifying device, and a method for automatic blood plasma purification. More particularly, this invention relates to a body fluid purifying material for effecting highly selective adsorptive separation of a pathogenic substance from a body fluid such as blood, a body fluid purifying device, and a method for automatic blood plasma purification.

[Background Art]

The lymphocyte is a cell differentiated from the hemocytic stem cell and endowed with an immunizing function. The lymphocytes are divided by function, character of cell membrane, etc. into a few subclasses. They are lymphocytic subclasses such as B lymphocytes which yield antibodies and fulfills humoral immunity, T lymphocytes which yield an immunity-regulating lymphokine, inflammatory lymphokine, etc. and fulfills cell-mediated immunity, and null-cells. The T and B lymphocytes further include functionally different subsets. In recent years, the desirability of a

technique for separating, refining, and concentrating such subclasses as T and B lymphocytes of different immunological properties in a pure form while minimizing infliction of functional damage thereon has been finding enthusiastic recognition in a wide range of social activities. In the basic studies of cytology and immunology, for example, the separation at least to the level of T and B lymphocytes is necessary for the in vitro analysis of in vivo immunizing systems. For the diagnosis, therapy, etc. in the clinical medicine, the transfusion or removal of specific fractions of lowered or heightened function is necessary. For the acquisition of lymphatic physiologically active substances, the separation and concentration of yielded cell fractions are important.

Heretofore, the separation of T lymphocytes and B lymphocytes has been generally effected by a procedure which comprises first obtaining a lymphocyte fraction from the peripheral blood or the vital tissues by the method of specific gravity centrifugation using such a high-density isotonic solution as a sodium diatrizoate-ficoll mixed solution and subsequently subjecting the lymphocyte fraction to a separating treatment thereby separating this fraction into T lymphocytes and B lymphocytes. The methods which are available for the subsequent separation treatment mentioned above are broadly divided into the three types, i.e. (1) methods aimed at specific antigens, receptors, and immunoglobulins on the surface of cell membranes, (2) methods for physical separation of cells, and (3) methods resorting to adsorption chromatography. The methods of the kind of (1) which are known to the art include a rosette forming method, a method of affinity chromatography (binding an antibody or immune complex), a cell lysis method using an antibody or a complement, a method of removal of mitogen activated lymphocytes, and a method resorting to a fluoresceine activated cell sorter, for example. These methods recover T lymphocytes or B lymphocytes in an intact

state only with difficulty because they are susceptible of strong bondage or stimulation of biologically high specificity. Moreover, many of these methods are unfit for mass treatment. The methods of the type of (2) which are known to the art include a method of density gradient centrifugation and a method of cell electrophoresis, for example. Since the T lymphocytes and the B lymphocytes have no notable difference in such physical properties as specific gravity and density, however, these methods cannot be expected to exhibit high accuracy in the yield or purity of separated and recovered cells. Among the methods of the type of (3) known to the art, the method which makes use of the difference the T lymphocytes and the B lymphocytes manifest in the specific adhesive ability relative to such extraneous substances as nylon, Tetoron, and cotton fibers is renowned. For the purpose of obtaining separated cells with high accuracy, this method requires an adsorption carrier to be treated as with fetal bovine serum (FBS) and entails the disadvantage that the operational conditions such as flow rate are not easily set. Moreover, this method suffers from heavy entrainment of impurities such as macrophage. These conventional methods generally involve complicate operations and necessitate troublesome preparations and have a serious disadvantage that experiences and technical skill are indispensable for operative assigned to the work of separation.

In recent years, therapy by replacement of blood plasma has been disseminating as a way of curing diseases in which specific components in body fluids induce serious symptoms. This therapy is particularly effective where a pathogenic substance is not easily dialyzed. It has been adapted suitably for the treatment of autoimmune diseases such as myasthenia gravis, serious arthritic rheumatism, and lupus erythematosus, diseases associated with immunity such as myoid nephritis, bronchial asthma, and multiple neuritis, rejection symptoms attendant on transplantation of internal

organs, liver failure, hypertension, and cancerous diseases. The term "autoimmune disease" as used herein refers to a disease which is caused in an individual by antibodies to be formed in consequence of humoral or cellular immune response to the individuals cells and tissues as antigen.

The therapy by replacement of blood plasma comprises indiscriminate removal of the whole blood plasma component and, therefore, entails the disadvantage that it not only expels such pathogenic substances as immunoglobulin, fibrinogen, and immune complex but also sacrifices albumin and other similar useful blood plasma components. To make the matter worse, it is pointed out that this therapy is fated to encounter numerous problems such as shortage of supply of blood plasma and blood plasma preparations as supplements, contraction of serum hepatitis and AIDS, complication of allergic diseases, and inducement of side effects.

A method for automatic blood plasma purification which effects selective removal of such macromolecular proteins as immunoglobulin, fibrinogen, and immune complex which constitute themselves pathogenic substances and returns automatic purifying blood plasma containing albumin and other similar useful blood plasma components to the patients circulatory system has been studied and put to use.

The absorbents which have found utility in this method of automatic blood plasma purification include porous resins (such as, for example, a styrene-divinylbenzene copolymer produced by Rohm and Haas Company and marketed under trademark designation of Amberlite XAD-4), ion exchangers (such as, for example, carboxymethyl cellulose and diethylaminoethyl agarose), inorganic porous substances (such as, for example, porous glass and ceramics), and affinity absorbents, for example. The conventional porous resins and ion exchangers not only possess small capacities for adsorption but also exhibit poor adsorptive specificity and, therefore, are suffered to adsorb albumin and other

similar useful components from the body fluid. When these absorbents are adopted for the aforementioned therapy, they not merely prevent the therapy from producing the effect thereof sufficiently but also throw some doubt on the safety of their use in the therapy as by inducing abnormal osmotic pressure peculiar to hypoproteinemia and consequent edema. The inorganic porous substances, though relatively satisfactory in terms of capacity for adsorption and adsorptive characteristic, have not yet been fully developed for practical use.

In contrast, the affinity adsorbents possess excellent capacity for adsorption and adsorptive specificity and, therefore, have a bright prospect of serving advantageously as a material for the purification of body fluid. The adsorbents of this type are divided by distinction in the action of adsorption into biological affinity adsorbents and physicochemical affinity adsorbents. The biological affinity adsorbents adsorb pathogenic substances in the body fluid by binding themselves with these pathogenic substances through such biological interactions as antigen-antibody fixation, complement fixation, and Fc fragment fixation. Thus, they are highly excellent in adsorptive specificity. Most of these adsorbents use such physiologically active macromlecular substances as DNA, anti-LDL antibody, and protein A as ligands (substances possessing affinity for pathogenic substances as targets) and, therefore, have the problem that the raw materials therefor are expensive and difficult of procurement. Further since the ligands are deficient in stability, it is difficult for these adsorbents and the columns packed therewith to retain activity intact through the steps of manufacture, sterilization, storage, transportation, and safekeeping. Moreover, since the ligands are inherently physiologically active substances, it must be taken into due consideration that the ligands, on contact with the blood, not only manifest their expected

affinity but also exhibit their inherent physiological actions and consequently induce side effects. Further, since the ligands are heterologous proteins, they are destined to induce side effects due to their antigenicity, when they are suffered to liberate themselves and exude from the adsorbents. The physicochemical affinity adsorbents serve the purpose of effecting adsorptive separation and removal of pathogenic substances in the body fluid by binding themselves with the pathogenic substances through such physical or chemical interactions as electrostatic bond and hydrophobic bond and so on. The ligands which are usable in this case include synthetic substances as polylysine, methylated albumin, tryptophan, and phenylalamine. These ligands are advantageous in that they are capable of being mass-produced, they are available at relatively low prices, and they exhibit activity stably. Most of them generally excel in the safety of contact with the blood. These adsorbents, therefore, are most promising materials for use in the aforementioned therapy. From the standpoint of their effects and prices, they are expected to find utility in the separation and purification of such specific humoral components as immunoglobulin, immune complex, and immunity-associated soluble factors and in the test of these components in addition to the utility as materials for the aforementioned therapy. The physicochemical affinity adsorbents which have been known to the art include porous substances possessing a carboxyl group or a sulfonic acid group on the surfaces thereof [Japanese Patent Laid-Open SHO 58(1983)-147,710, SHO 57(1982)-56,038, SHO 57(1982)-75,141, SHO 57(1982)-170,263, and SHO 57(1982)-197,294], hydrophilic carriers having a hydrophobic amino acid bonded thereto [Japanese Patent Laid-Open SHO 57(1982)-122,875, SHO 58(1983)-15,924, SHO 58(1983)-165,859, and SHO 58(1983)-165,861], hydrophilic carriers having a modified immunoglobulin (IgG) bonded thereto [Japanese Patent Laid-Open SHO 57(19829-77,614, SHO

57(1982)-77,625, and SHO 57(1982)-156,035], porous substances having methylated albumin bonded thereto [Japanese Patent Laid-Open SHO 55(1980)-120,875 and SHO 55(1980)-125,872], hydrophilic carriers having sugar bonded thereto [Japanese Patent Laid-Open SHO 57(1982)-134,164 and SHO 58(1983)-133,257], porous substances having urine base, pyrimidine base, or a sugar phosphate bonded thereto [Japanese Patent Laid-Open SHO 57(1982)-192,560, SHO 58(1983)-61,752, and SHO 58(1983)-98,142], and insoluble carriers having bonded thereto a peptide possessing affinity for sugar chain [Japanese Patent Laid-Open SHO 60(2985)-163,667], for example.

It is not just to evaluate these conventional physicochemical affinity adsorbents as being fully capable of fitting the treatment of the autoimmune diseasen by the aforementioned therapy of automatic blood plasma purification. In the circumstances, the desirability of developing a purifying material and a purifying device capable of removing pathogenic substances with high adsorption efficiency and high adsorptive specificity while restraining the inevitable infliction of adverse effects on the body fluid and a method for automatic blood plasma purification using the purifying material and the purifying device has been finding growing recognition.

This invention, therefore, has an object of providing a novel B lymphocyte separating material, a method for the separation of B lymphocytes, and a separating device therefor. This invention also discloses a B lymphocyte separating material capable of effecting the separation expediently and inexpensively in high yield with high purity without adversely affecting the activities of cells, a method for the separation, and a separating device therefor. This invention has a further object of providing a B lymphocyte separating material, a method for the separation, and a separating device thereof which fit volume treatments.

Another object of this invention is to provide a novel body fluid purifying material, a body fluid purifying device, and a method for automatic blood plasma purification. Still another object of this invention is to provide a body fluid purifying material and a body fluid purifying device capable of effecting adsorptive removal of pathogenic substances with a high capacity for adsorption, high adsorptive specificity, and with high safety from the body fluid such as the blood and a method for automatic blood plasma purification by the use of the material and the device mentioned above. Yet another object of this invention is to provide a body fluid purifying material and a body fluid purifying device possessing stable qualities and consequently easily tolerating the impacts of sterilization, storage, transportation, and safekeeping and a method for automatic blood plasma purification by the use of the material and the device mentioned above. This invention has a further object of providing a body fluid purifying material and a body fluid purifying device available at low prices and capable of mass production and a method for automatic blood plasma purification by the use of the material and the device mentioned above.

[Disclosure of Invention]

The objects described above are accomplished by a B lymphocyte separating material, characterized by the fact that an organically synthesized ligand possessing affinity for immunoglobulin is fixed on the surface of a water-insoluble solid substance.

This invention also discloses a B lymphocyte separating material, wherein the organically synthesized ligand is selected from the group consisting of heterocyclic compounds, peptides, and amino acids. This invention further discloses a B lymphocyte separating material, wherein the heterocyclic compound is selected from the group consisting of chemotherapeutic agents and derivatives thereof, pyrimidine-associated compounds, pyridine-associated compounds, and pyrazine-associated compounds. This invention further discloses a B lymphocyte separating material, wherein the heterocyclic compound is selected from the group consisting of sulfa drugs, compounds formed of sulfa drugs with coloring matters of pK 4.0 to 9.0, 2-thiouracil, isonicotinic acid hydrazide, and luminol. This invention further discloses a B lymphocyte separating material, wherein the peptide is an oligopeptide having the number of amino acids approximately in the range of 2 to 10. This invention further discloses a B lymphocyte separating material, wherein the oligopeptide consists of lysine, and tyrosine, or an aspartylphenylalanine alkyl ester. This invention further discloses a B lymphocyte separating material, wherein the amino acid is one amino acid or a combination of two or more amino acids selected from the group consisting of valine, leucine, tyrosine, phenylalamine, isoleucine, tryptophan, lysine, arginine, proline, and aspartic acid, and derivatives thereof. This invention further discloses a B lymphocyte separating material, wherein the organically synthesized ligand is selected formed by the combination of arginine, aspartic acid, phenylalamine, and proline with sulfathiazole. This invention further discloses a B lymphocyte separating material, wherein the water-insoluble solid substance comprises a synthetic organic macromolecular substance, a natural organic macromolecular substance, or an inorganic substance. This invention further discloses a B lymphocyte separating material, wherein the water-insoluble solid substance comprises particles possessing an average particle diameter in the range of 0.05 to 5 mm. This invention further discloses a B lymphocyte separating material, wherein the organically synthesized ligand is fixed by a coralent bond on the surface of the water-insoluble solid substance.

The various objects described above are also accomplihsed by a method for the separation of B

lymphocytes, characterized by the fact that a B lymphocyte suspension liquid is caused to contact a separating material obtained by fixing on the surface of a water-insoluble solid substance an organically synthesized ligand possessing affinity for immunoglobulin so as to effect selective separation of B lymphocytes possessing an immunoglobulin molecule (S-Ig) on the membrane surface.

This invention also discloses a method for the separation of B lymphocytes, wherein the ligand fixed on the separating material is selected from the group consisting of heterocyclic compounds, peptides, and amino acids. This invention further discloses a method for the separation of B lymphocytes, wherein the heterocyclic compound is selected from the group consisting of chemotherapeutie agents and derivatives thereof, pyrimidine-associated compounds, pyridine-associated compounds, and pyrazine-associated compounds. This invention further discloses a method for the separation of B lymphocytes, wherein the heterocyclic compound is selected from the group consisting of sulfa drugs, compounds formed of sulfa drugs with coloring matters of pK 4.0 to 9.0, 2-thiouracil, isonicotinic acid hydrazide, and luminol. This invention further discloses a method for the separation of B lymphocytes, wherein the peptide is an oligopeptide having the number of amino acids approximately in the range of 2 to 10. This invention further discloses a method for the separation of B lymphocytes, wherein oligopeptide consists of and tyrosine, or an aspartylphenylalanine alkyl ester. This invention further discloses a method for the separation of B lymphocytes, wherein the amino acid is one amino acid or a combination of two or more amino acids selected from the group consisting of valine, leucine, tyrosine, phenylalanine, isoleucine, tryptophan, lysine, arginine, proline, and aspartic acid, and derivatives thereof. This invention further discloses a method for the separation of B lymphocytes, wherein the ligand fixed on the separating material is formed by the

combination of arginine, aspartic acid, phenylalanine, and proline with sulfathiazole. This invention further discloses a method for the separation of B lymphocytes, wherein the water-insoluble solid substance comprises a synthetic organic macromolecular substance, a natural organic macromolecular substance, or an inorganic substance. This invention further discloses a method for the separation of B lymphocytes, wherein an isotonic buffer solution is used as a rinsing solution. This invention further discloses a method for the separation of B lymphocytes, wherein the separating material is treated with bovine serum albumin prior to the contact thereof with the lymphocyte suspension.

The various objects described above are further accomplihsed by a B lymphocyte separating device, characterized by comprising a column packed with a separating material obtained by fixing on the surface of a water-insoluble solid substance an organically synthesized ligand possessing affinity for immunoglobulin.

This invention further discloses a B lymphocyte separating device, wherein the column is made of polypropylene or polycarbonate. This invention further discloses a B lymphocyte separating device, wherein the column is provided between the inlet/outlet thereof and the separating material bed thereof with a filter possessing meshes pervious to the cell component of the B lymphocyte-containing liquid and impervious to the separating material. This invention further discloses a B lymphocyte separating device, wherein the filter is made of polyester or polyamide.

The various objects described above are further accomplished by a B lymphocyte separating material, characterized by the fact that an organically synthesized ligand possessing affinity for immunoglobulin is fixed on the surface of a water-insoluble solid substance through the medium of a spacer. This invention further discloses a B

lymphocyte separating material, wherein the spacer has as the skeleton thereof a hydrocarbon chain possessing at least two carbon atoms or a hydrocarbon chain containing a characteristic group. This invention further discloses a B lymphocyte separating material, wherein the repetition of methylene in the spacer has as the skeleton thereof at least four linear alkyls. This invention further discloses a B lymphocyte separating material, wherein the spacer possesses an ethylene oxide skeleton having a polymerization degree in the range of 1 to 50. This invention further discloses a B lymphocyte separating material, wherein the organically synthesized ligand is selected from the group consisting of heterocyclic compounds, peptides, and amino acids. This invention further discloses a B lymphocyte separating material, wherein the heterocyclic compound is selected from the group consisting of chemotherapeutic agents and derivatives thereof, pyrimidine-associated compounds, pyridine-associated compounds, and pyrazine-associated compounds. This invention further discloses a B lymphocyte separating material, wherein the heterocyclic compound is selected from the group consisting of sulfa drugs, compounds formed of sulfa drugs with coloring matters of pK 4.0 to 9.0, 2-thiouracil, isonicotinic acid hydrazide, and luminol. This invention further discloses a B lymphocyte separating material, wherein the peptide is an oligopeptide having the number of amino acids approximately in the range of 2 to 10. This invention further discloses a B lymphocyte separating material, wherein the oligopeptide consists of lysine, and tyrosine, or an aspartylphenylalanine alkyl ester. This invention further discloses a B lymphocyte separating material, wherein the amino acid is one amino acid or a combination of two or more amino acids selected from the group consisting of valine, leucine, tyrosine, phenylalanine, isoleucine, tryptophan, lysine, arginine, proline, and aspartic acid, and derivatives thereof. This invention further discloses a B lymphocyte separating

material, wherein the organically synthesized ligand is selected formed by the combination of arginine, aspartic acid, phenylalanine, and proline with sulfathiazole. This invention further discloses a B lymphocyte separating material, wherein the water-insoluble solid substance comprises a synthetic organic macromolecular substance, a natural organic macromolecular substance, or an inorganic substance. This invention further discloses a B lymphocyte separating material, wherein the water-insoluble solid substance comprises particles possessing an average particle diameter in the range of 0.05 to 5 mm.

The various objects described above are further accomplished by a method for the separation of a B lymphocytes, characterized by the fact that a B lymphocyte suspension liquid is caused to contact a separating material obtained by fixing on the surface of a water-insoluble solid substance through the medium of a spacer an organically synthesized ligand possessing affinity for immune globulin so as to effect selective sequestration of B lymphocytes possessing an immune globulin molecule (S-Ig) on the membrane surface.

This invention further discloses a method for the separation of B lymphocytes, wherein the spacer in the separating material has as the skeleton thereof a hydrocarbon chain possessing at least two carbon atoms or a hydrocarbon chain containing a characteristic group. This invention further discloses a method for the separation of B lymphocytes, wherein the repetition of methylene in the spacer has as the skeleton thereof at least four linear alkyls. This invention further discloses a method for the separation of B lymphocytes, wherein the spacer in the separating material possesses an ethylene oxide skeleton having a polymerization degree in the range of 1 to 50. This invention further discloses a method for the separation of B lymphocytes, wherein the ligand fixed on the separating material is selected from the group consisting of

hererocyclic compounds, peptides, and amino acids. This invention further discloses a method for the separation of B lymphocytes, wherein the heterocyclic compound is selected from the group consisting of chemotherapeutic agents and derivatives thereof, pyrimidine-associated compounds, pyridine-associated compounds, and pyrazine-associated compounds. This invention further discloses a method for the separation of B lymphocytes, wherein the heterocyclic compound is selected from the group consisting of sulfa drugs, compounds formed of sulfa drugs with coloring of pK 4.0 to 9.0, 2-thiouracil, isonicotinic acid hydrazide, and luminol. This invention further discloses a method for the separation of B lymphocytes, wherein the peptide is an oligopeptide having the number of amino acids approximately in the range of 2 to 10. This invention further discloses a method for the separation of B lymphocytes, wherein the oligopeptide consists of lysine, and tyrosine, or an aspartylphenylalamine alkyl ester. This invention further discloses a method for the separation of B lymphocytes, wherein the amino acid is one amino acid or a combination of two or more amino acids selected from the group consisting of valine, leucine, tyrosine, phenylalanine, isoleucine, tryptophan, lysine, argining, proline, and aspartic acid, and derivatives thereof. This invention further discloses a method for the separation of B lymphocytes, wherein the ligand fixed on the separating material is formed by the combination of arginine, aspartic acid, phenylalanine, and proline with sulfathiazole. This invention further discloses a method for the separation of B lymphocytes, wherein the water-insoluble solid substance comprises a synthetic organic macromolecular substance, a natural organic macromolecular substance, or an inorganic substance. This invention further discloses a method for the separation of B lymphocytes, wherein an isotonic buffer solution is used as the rinsing solution.

The various objects described above are further accomplished by a B lymphocyte separating device, characterized by comprising a column packed with a separating material obtained by fixing on the surface of a water-insoluble solid substance through the medium of a spacer an organically synthesized ligand possessing affinity for immunoglobulins.

This invention further discloses a B lymphocyte separating device, wherein the column is made of polypropylene or polycarbonate. This invention further discloses a B lymphocyte separating device, wherein the column is provided between the inlet/outlet thereof and the separating material bed thereof with a filter possessing meshes pervious to the cell component of the B lymphocyte suspension and impervious to the separating material. This invention further discloses a B lymphocyte separating device, wherein the filter is made of polyester or polyamide.

The various objects described above are further accomplished by a body fluid purifying material, characterized by having a dipeptide or a derivative thereof fixed on the surface of a water-insoluble carrier.

This invention further discloses a body fluid purifying material, wherein the dipeptide or the derivative thereof is a dipeptide of an acidic amino acid with an aromatic amino acid or a heterocyclic amino acid or a derivative thereof. This invention further discloses a body fluid purifying material, wherein the dipeptide or the derivative thereof is a dipeptide of aspartic acid or glutamic acid with phenylalanine, tyrosine, tryptophan, proline, or hydroxyproline or a derivative thereof. This invention further discloses a body fluid purifying material, wherein the dipeptide or the derivative thereof is aspartylphenylalanine or a derivative thereof. This invention further discloses a body fluid purifying material, wherein the dipeptide or the derivative thereof is an

aspartylphenylalanine methyl ester. This invention further discloses a body fluid purifying material, wherein the water-insoluble carrier is a particulate carrier. This invention further discloses a body fluid purifying material, wherein the particulate carrier possesses an average particle diamter in the range of 0.05 to 5 mm. This invention further discloses a body fluid purifying material, wherein the water-insoluble carrier is a porous substance. This invention further discloses a body fluid purifying material, wherein the water-insoluble carrier is made of a synthetic organic macromolecular substance, a natural organic macromolecular substance, or an inorganic substance. This invention further discloses a body fluid purifying material, wherein the water-insoluble carrier comprises porous acrylic ester type particles or porous chitosan particles. This invention further discloses a body fluid purifying material, wherein the dispeptide or the derivative thereof is fixed by a covalent bond to the surface of the water-insoluble carrier. This invention further discloses a body fluid purifying material, wherein the dipeptide or the derivative thereof is fixed by covalent bond through the medium of a spacer to the surface of the water-insoluble carrier.

The various objects described above are further accomplished by a body fluid purifying device, characterized by comprising a column packed with a purifying material having a dipeptide or a derivative thereof fixed on the surface of a water-insoluble carrier.

This invention further discloses a body fluid purifying device, which has undergone a wet-hot sterilizing treatment. This invention further discloses a body fluid purifying device, wherein the purifying material has a dipeptide or a derivative thereof fixed on the surface of the water-insoluble carrier. This invention further discloses a body fluid purifying device, wherein the column is made of polypropylene or polycarbonate. This invention

further discloses a body fluid purifying device, wherein the column is provided between the inlet/outlet thereof and the purifying material bed with a filter possessing meshes pervious to the body fluid component and impervious to the purifying material. This invention further discloses a body fluid purifying device, wherein the dipeptide or the derivative thereof is a dipeptide of an acidic amino acid and an aromatic cyclic amino acid or a heterocyclic amino acid or a derivative thereof. This invention further discloses a body fluid purifying device, wherein the dipeptide or the derivative thereof is a dipeptide of aspartic acid or glutamic acid with phenylalanine, tyrosine, tryptophan, proline, or hydroxyproline or a derivative thereof. This invention further discloses a body fluid purifying device, wherein the dipeptide or the derivative thereof is aspartylphenylalanine or a derivative thereof. This invention further discloses a body fluid purifying device, wherein the dipeptide or the derivative thereof is an aspartylphenylalanine methyl ester. This invention further discloses a body fluid purifying device, wherein the particulate carrier possesses an average particle diameter in the range of 0.05 to 5 mm. This invention further discloses a body fluid purifying device, wherein the water-insoluble carrier is a particulate carrier. This invention further discloses a body fluid purifying device, therein the water-insoluble carrier is made of a synthetic organic macromolecular substance, a natural organic macromolecular substance, or an inorganic substance. This invention further discloses a body fluid purifying device, wherein the water-insoluble carrier comprises porous acrylic ester type particles or porous chitosan particles. This invention further discloses a body fluid purifying device, wherein the dipeptide or the derivative thereof is fixed by a covalent bond to the surface of the water-insoluble carrier. This invention further discloses a body fluid purifying device, wherein the dipeptide or the derivative thereof is fixed by

a covalent bond through the medium of a spacer to the surface of the water-insoluble carrier.

The various objects described above are further accomplished by a method for automatic blood plasma purification, comprising the steps of separating the blood led out of a patient into a blood component and a blood plasma component, causing the blood plasma component to contact a body fluid purifying material characterized by having a dipeptide or a derivative thereof fixed on the surface of a water-insoluble carrier, causing the blood plasma component resulting from the treatment with the body fluid purifying material to be mixed with the blood component, and returning the resultant mixture to the patient.

This invention further discloses a method for automatic blood plasma purification, wherein the dipeptide or the derivative thereof is a dipeptide of an acidic amino acid with an aromatic cyclic amino acid or a heterocyclic amino acid or a derivative thereof. This invention further discloses a method for automatic blood plasma purification, wherein the dipeptide or the derivative thereof is a dipeptide of aspartic acid or glutamic acid with phenylalanine, tyrosine, tryptophan, proline, or hydroxyproline or a derivative thereof. This invention further discloses a method for automatic blood plasma purification, wherein the dipeptide or the derivative thereof is aspartylphenylalanine or a derivative thereof. This invention further discloses a method for automatic blood plasma purification, wherein the dipeptide or the derivative thereof is an aspartylphenylalanine methyl ester. This invention further discloses a method for automatic blood plasma purification, wherein the water-insoluble carrier is a particulate carrier. This invention further discloses a method for automatic blood plasma purification, wherein the particulate carrier possesses an average particle diameter in the range of 0.5 to 5 mm. This

invention further discloses a method for automatic blood plasma purification, wherein the water-insoluble carrier is a porous substance. This invention further discloses a method for automatic blood plasma purification, wherein the water-insoluble carrier is made of a synthetic organic macromolecular substance, a natural organic macromolecular substance, or an inorganic substance. This invention further discloses a method for automatic blood plasma purification, wherein the water-insoluble carrier comprises porous arylic ester type particles or porous chitosan particles. This invention further discloses a method for automatic blood plasma purification, wherein the dipeptide or the derivative thereof is fixed by a covalent bond to the surface of the water-insoluble carrier. This invention further discloses a method for automatic blood plasma purification, wherein the dipeptide or the derivative thereof is fixed by a covalent bond through the medium of a spacer to the surface of the water-insoluble carrier.

The term "ligand" as used in this specification refers to a substance which binds itself specifically with a protein.

The term "pathogenic substance" as substance subjected to the adsorption with the body fluid purifying material and the body fluid purifying device of the present invention, is a harmful protein contained in the body fluid. The pathogenic substances which answer the description include autoantibodies such as ordinary immunoglobulins (IgA, IgD, IgE, IgG, and IgM), rheumafoid factors, antinucleic antibody anti-DNA antibody, anti-lymphocyte antibody, anti-red blood cell antibody, anti-platelet antibody, anti-acetylcholine receptor antibody, anti-colon antibody, antiglomerulus antibody, pulmonary basement membrane antibody, antidermal spinal intercellular desmosome antibody, anti-insulin receptor antibody, anti-myelin antibody, and anti-hemophilia VIII factor antibody, reduction products of immunoglobulin, complexes between

immunoglobulins or between immunoglubulin and other substance (particularly an antigen or an antigen-like substance), lymphokine, complements, and fibrinogen, for example.

[Brief Description of Drawings]

Fig. 1 is a model diagram illustrating a typical manner of using a B-lymphocyte separating device as one embodiment of this invention. Fig. 2 is a cross section illustrating in model a typical body fluid purifying device as one embodiment of this invention. Fig. 3 is a circuit diagram illustrating a method of extracorporeal circulation therapy incorporating therein a body fluid purifying device of this invention.

[Best Mode for Carrying Out the Invention]

As widely known, immunoglobulins of varying classes such as IgG, IgM, and IgA, occur on the surface of cellular membrane of the B lymphocytes, depending on the degree of differentiation. It is further known that on the surface of cellular membrane of T lymphocytes, these immunoglobulins do not substantially occur.

Incidentally, the B lymphocyte separating material of this invention is characterized by having fixed on the surface of a water-insoluble solid substance an organically synthesized ligand possessing affinity for immunoglobulin. When this B lymphocyte separating material is allowed to contact a B lymphocyte suspension, it manifests extremely high affinity for the B lymphocites which possess immunoglobulins on the surface of cellular membrane thereof, with the result that the B lymphocytes will be capturted by the B lymphocyte separating material with extremely high probability. In contrast, the B lymphocyte separating material exhibits no affinity for the T lymphocytes having no immunoglobulin on the surface of cellular membrane thereof, with the result that the T lymphocytes will not be sequestered by the B lymphocyte separating material. By separating the adherent fraction of the separating material and the non-adherent

fraction of the separating material from each other, therefore, the B lymphocytes and the T lymphocytes can be obtained with high purity in high yield.

Another B lymphocyte separating material of this invention is characterized by having fixed on the surface of a water-insoluble solid substance through the medium of a spacer an organically synthesized ligand possessing high affinity for immunoglobulin. When this B lymphocyte separating material is caused to contact a lymphocyte suspension, for example, it exhibits extremely high affinity for the B lymphocytes possessing immunoglobulin on the surface of cellular membrane thereof and, therefore, the B lymphocytes are captured by the B lymphocyte separating material with extremely high probability. In contrast, the B lymphocyte separating material exhibits no affinity for the T lymphocytes possessing no immunoglobulin on the surface of cellular membrane thereof. Further, the spacer chains which interposed between the surface of the water-insoluble solid substance and the organically synthesized ligand possessing high affinity for immunoglobulin, owing to the motion or exclude volume effect thereof, prevents cells from approaching the surface of the separating material and causing interaction therewith and consequently non-specific adhesion of T lymphocytes which possesses a weak interacting force to the organically synthesized ligand was reduced preferentially compared with B lymphocyte as described above. Thus, the T lymphocytes are not substantially captured by the B lymphocyte separating material. By separating the adherent fraction of the separating material and the non-adherent fraction of the separating material from each other, therefore, the B lymphocytes and the T lymphocytes can be obtained with high purity in high yield.

By preparing a column packed with the B lymphocyte separating material and provided with an outlet and an inlet, pouring a lymphocyte suspension at a constant rate of

flow into the column, and immediately washing the non-adherent fraction out of the column interior with a washing liquid such as an isotonic buffer solution, therefore, the T lymphocytes can be conveniently and quickly obtained in a large amount with high purity in high yield. Though this invention utilizes one form of the technique of adsorption chromatography, the T lymphocytes and the B lymphocyes are separated in their intact state because the B lymphocyte separating material of this invention, unlike the separating material in the conventional affinity chromatography which has an antibody and a immune complex immobilized on a carrier, is characterized by possessing a character of imparting absolutely no stimulation of biologically high specificity. The affinity of this nature exhibited by the B lymphocyte separating material of this invention is confirmed to have no effect on any origin of animal species. The operation of the separation is completed in several minutes. The lymphocyte suspension has only to rely for its composition on a simple isotonic salt solution and has no use for a concentrated serum originating in an animal of different species such as, for example, bovine fetal serum. Thus, the B lymphocyte separating material enjoys many advantages from the standpoint of practical use.

The organically synthesized ligand possessing high affinity for immunoglobulin and used in the B lymphocyte separating material as described above is inferred to possess high affinity also for the aforementioned pathogenic substances (including immunoglobulins) in the body fluid. In the peptides counted among the compounds known to be highly suitable as ligands in the B lymphocyte separating material, the dipeptides or the derivatives thereof have been demonstrated to be highly useful for the selective adsorption of the pathogenic substances. This knowledge has culminated into another aspect of this invention.

To be specific, the body fluid purifying materail of this invention is one kind of a physicochemical affinity

adsorbent characterized by having a dipeptide or a derivative thereof fixed on the surface of a water-insoluble carrier. As demonstrated in working examples to be cited hereinafter, it manifests extremely high adsorption efficiency and adsorption specificity to the pathogenic substances.

Generally, a purifying material formed solely of a hydrophobic compound, namely a purifying material of strong hydrophobic bond is unfit for the selective removal of pathogenic substances because it adsorbs not only pathogenic substances but also the albumin fraction and other similar useful proteins in the body fluid. In contrast, a purifying material formed of a hydrophilic compound of strong hydrogen bond or a compound abounding with charging groups of strong electrostatic bond is incapable of sufficiently adsorbing pathogenic substances because it has a very small capacity for adsorption of proteins. These points may well imply that a purifying material properly combining various interactions is fit for selective separation and removal of pathogenic substances from the body fluid with high adsorption efficiency and adsorption specificity.

The body fluid purifying material of this invention achieves desirable in the adsorption of the aforementioned pathogenic substances, particularly immunoglobulin or immunoglobulin-associated compounds (hereinafter referred to as immunoglobulin type compounds). This fact may be logically explained by a supposition that the hydrophobicity and the electrostatic property owned by the purifying material manifest interactive forces with the amino acid residues of the adsorbed parts of the compounds subjected to adsorption (immunoglobulin type compounds) and the steric structure of the surface of the purifying material properly first into the intramolecular pockets of the compounds subjected to adsorption (immunoglobulin type compounds). Moreover, the compound to be used as a ligand in the body fluid purifying material of this invention is a dipeptide.

The peptide, unlike the physiologically active macromolecular substance used in the conventional biological affinity adsorbent, is easily synthesized or procured at a relatively low price and is excellent in stability. Thus, the body fluid purifying material and the body fluid purifying device contemplated by the present invention are very easy invariably of production, sterilization, storage, transportation, and safekeeping. In the state undergoing any of these actions, the purifying material has the activity neither degraded nor lost. As concerns the safety of the purifying material for contact with the body fluid, the purifying material warrants very high safety even if it should liberate the dipeptide as the ligand on contact with the body fluid, because the dipeptide is never allowed to manifest any physiological action in the body fluid but is easily decomposed and consequently metabolized as amino acid.

Now, the present invention will be described more specifically below with reference to working examples. For the purpose of facilitating comprehension of this invention, the sections titled "B Lymphocyte Separation Material I", "B Lymphocyte Separation Material II", "B Lymphocyte Separating Method and Separating Device", "Body Fluid Purifying Material", "Body Fluid Purifying Device", "Method for Automatic Blood Plasma Purification", and "Examples" will be incorporated in the following text of the specification.

## B lymphocyte Separating Material I

The B lymphocyte Separating Material of the present invention is characterized by having fixed on the surface of a water-insoluble solid substance an organically synthesized ligand possessing high affinity for immunoglobulin. The ligands which possess high affinity for immunoglobulin include heterocyclic compounds, peptides, and amino acids, for example. They may be used either singly or in the form of a combination of two or more members as a ligand.

Specifically, the heterocyclic compounds which are usable in the present invention include five-member heterocyclic compounds such as furan and derivatives thereof, thiophene and derivatives thereof and dithiolan derivatives, and azoles; six-member heterocyclic compounds such as pyridine and associated compounds thereof, quinoline and associated compounds thereof, acridine and associated compounds thereof, pyrimidine and associated compounds thereof, and pyran and pyrone and associated compounds thereof; condensed heterocyclic compounds such as phenoxazine, phenothiazine, pterin, alloxazine compounds; porphyrin type heterocyclic compounds such as purine base, nucleic acid, hemin, chlorophyl, vitamin B , and phthalocyanine; and alkaloids. Among other heterocyclic compounds cited above, chemotheraputics and derivatives thereof, pyrimidine-associated compounds, pyridine-associated compounds, and pyrazine-associated compounds, etc. produce particularly desirable results and sulfa drugs, compounds of sulfa drugs with coloring matters of pK 4.0 to 9.0, 2-thiouracil, isonicotinic acid hydrazide, and luminol afford still better results. The expression "coloring matters of pK 4.0 to 9.0" as used herein refers to those of the various indicators and histochemical grade coloring matters indicated in H. J. Conn's Biological Stains, R. D. Little, The Willians & Wilkins Company, Baltimore, 1977 which have pK values in the range of 4.0 to 9.0. The coloring matters are preferable to possess pK values roughly equaling the isoelectric points of immunoglobulins. Specifically, such pK values fall in the range of 4.0 to 9.0. Among other sulfa drugs and compounds of sulfa drugs and coloring matters of pK values of 4.0 to 9.0, those using sulfathiazole, sulfamethizole, and soulfisomezole as sulfa drugs and lacmoid, brilliant yellow, and phenol red as coloring matters of pK 4.0 to 9.0 achieve particularly desirable results. These sulfa drugs are $N^{-1}$-heterocyclic

compounds whose heterocycles belong to the azole class, i.e. thiazole, 2-methylthiazole, and 2-methylisooxazole.

The hetero atoms of pyridines, pyrimidines, and azoles which are heterocyclic compounds advantageously usable as ligands of high affinity for immunoglobulin in the B lymphocyte separating material of the present invention possess solitary electron pairs and function as proton acceptors. The heterocycles destitute of dissociation groups exhibit hydrophobicity. Further, the sulfonamide part of sulfa drugs, the thiol group or hydroxyl group of 2-thuiouracil, the acid amide part of isonicotinic acid hydrazide, and the carbonyl group and the imino group of luminol possess an ability to form a hydrogen bond, In the interaction between the immunoglobulin on the surface of cellular membrane of the B lymphocyte and the compound under discussion, the hydrophobicity of the principal part of the compound, the electrostatic characteristic due to the hetero atom, and the hydrogen bond property near the principal part severally manifest an interactive force with the amino acid residue at the adsorption site in the molecular chain of the immunoglobulin. This interactive force coupled with the fact that the steric structure of the compound properly fits in the intramolecular pocket of the immunoglobulin on the surface of the cellular membrane is believed to give rise to the high affinity of the non-antigen/antibody system.

The peptide to be used in the present invention is a synthetic compound which is obtained by the union of at least two amino acids through the medium of a peptide bond. Among other peptides answering the description, oligopeptides having a number of amino acids approximately in the range of 2 to 10 affords desirable results. Among these oligopeptides, those which simulate the affinity side of such known natural substances as protein A possessing a specific ability to form a bond with the molecule of immunoglobulin prove to be particularly desirable. Especially such compounds as lysine, oligopeptides

originating in tyrosine, and aspartylphenylalanine alkyl esters such as aspartylphenylalanine methyl esters produce unusually desirable results. These peptides are thought to acquire high affinity of the non-antigen/antibody system owing to the interaction of the specific hydrophobic site and the charging site in the molecular chain of immunoglobulin.

The amino acids which are usable in this invention may be used either singly or in the form of two or more members. To be specific, one member or a varying combination of two or more members selected from the group consisting of hydrophobic valine, leucine, tyrosine, phenylalanine, isoleucine, and tryptophan, basic lysine and arginine, and acidic proline and aspartic acid, and derivatives thereof produces particularly desirable results. The distinction between the D- and L-form of these substances is not critical.

Similarly to the peptides previously mentioned, the amino acids just mentioned are thought to acquire high affinity of the non-antigen/antibody system for the B lymphocyte possessing immunoglobulin owing to the interaction with the specific hydrophobic site and the charging site in the molecular of the immunoglobulin.

Further, the organically synthesized ligand described above is capable of fully manifesting the affinity for the B lymphocyte even when it is used alone. Particularly when an amino acid such as arginine, aspartic acid, phenylalanine, or proline is used in combination with sulfathiazole, a heterocyclic compound, as a ligand, there are obtained unusually satisfactory results. The ligands which are enumerated above exhibit high affinity for the immunoglobuline originating in human, bovine, and rat. Thus, the possibility is high that the particularity of the species of animal as the source for the cells subjected to the treatment of purification is not critical.

In the B lymphocyte separating material of this invention, the water-insoluble solid substances which are usable as the carrier for fixing thereon the organically synthesized ligand of the nature described above include organic macromolecular compounds of agarose type, dextran type, cellulose type, polyacrylamide type, polyvinyl alcohol type, polyvinyl pyrrolidone type, polyacrylonitrile type, styrene-divinylbenzene copolymer type, polystyrene type, acrylic ester type, methacrylic ester type, polyethylene type, polypropylene type, polyethylene 4-fluoride type, ethylene-vinyl acetate copolymer type, polyamide type, polycarbonate type, polyvinylidene fluoride type, polyvinylformal type, polyarylate type, and polyether sulfone type, and inorganic substances of glass type, alumina type, titanium type, activated carbon type, and ceramic type, for example. In these substances, those which have no unduly high ability of cell adhesion, namely which produces relatively moderate interaction between cells and carrier, are particularly desirable. The organic compounds of the acrylic ester type are used particularly advantageously. Such natural organic macromolecular substances of biotic origin as collagen and chitosan are also usable. The shape in which the water-insoluble solid substance is used herein is not specifically defined. The water-insoluble solid substance may be used in the shape of a flat plate. It is particularly desirable to use this substance in the form of particles having an average diameter in the range of 0.05 to 5 mm. The average particle diameter as mentioned herein is what is determined by classifying a given sample of particles with sieves defined in Japanese Industrial Standard (JIS) Z-8801, registering the intermediate value between the upper limit and the lower limit of particle diameters in each of the classes, and averaging the weights of these intermediates representing the relevant classes. The individual particles of the water-insoluble solid substance are desired to be in a

spherical shape in the sense that such particles do not readily inflict physical damage to the cells and they are produced easily with high uniformity of size.

In the B lymphocyte separating material of this invention, the fixation of the organically synthesized ligand on the surface of the water-insoluble solid substance of the quality mentioned above may be attained by any of virtually all of the known methods such as covalent bond, ion bond, physical adsorption, wrapping, and precipitative insolubilization on the surface of carrier, for example. In view of the fixed ligandś ability to dissolve out, it is desirable for the ligand to be used as fixed and insolubilized by covalent bond on the surface of the carrier. For this purpose, therefore, the method for activation of an insolubilized carrier and the method for fixation of a ligand which are well known and used in the field of immobilized enzymes and affinity chromatography can be advantageously used.

Lymphocyte Separating Material II

The other B lymphocyte separating material of this invention is characterized by having fixed on the surface of a water-insoluble solid substance through the medium of a spacer an organically synthetized ligand possessing affinity for immunoglobulin. The ligands possessing high affinity for immunogloubulin and used in the B lymphocyte separating material are the same as those cited previously as usable in the former B lymphocyte separating material. They have the same functions as described above. The water-soluble solid substances which are usable as the carrier for fixing the organically synthesized ligand are the same as those cited previously.

The spacer to be used in the other B lymphocyte separating material of this invention is required only to assume a molecular form and to be capable of being interposed as extended in a desired length between the surface of the water-insoluble solid substance and the

ligand. It may possess a skeleton structure of any sort on the condition that the skeleton structure is incapable of causing a steric hindrance of the bond between the surface of the water-insoluble solid substance and the ligand. When the spacer of this nature intervenes between the surface of the water-insoluble solid substance and the ligand, the cells are prevented from approaching the surface of the separating material and producing an interaction therewith by the flowability and the volume-expelling effect of the spacer, with the possible result that the adhesion of the T lymphocytes possessing a weak interactive force with the separating material is preferentially curbed. Thus, the intervening spacer brings about an effect of curbing the non-specific adhesion of the T lymphocytes.

This spacer has as its skeleton a hydrophobic hydrocarbon chain possessing at least two carbon atoms or a hydrophilic hydrocarbon chain containing in the molecular chain thereof such a characteristic group as hydroxyl group, carboxyl group, or ether oxygen. It is also such that the known reactive functional groups such as epoxy groups or isothiocyanate groups which the spacer possesses one each at the opposite terminals of the molecular chain are caused to react respectively with the surface of the water-insoluble substance and the amino group or carboxyl group of the ligand and consequently one end of the molecular chain of the spacer is attached to the surface of the water-insoluble substance and the other end thereof to the ligand each by covalent bond.

The hydrophobic hydrocarbon chains which the spacer is allowed to have as its skeleton structure include alkyl chains formed of simple repetition of methylene, such alkyl chains partially containing a carbon double bond or a various cyclic substituent such as a ring, and such molecular chains as mentioned above which contain branches, for example. Among other hydrophobic hydrocarbon chains mentioned above, linear alkyl chains possessing at least

four repeated methylenes, preferably 4 to 10 repeated methylenes, prove to be particularly desirable in that they permit the spacer to manifest a notably high effect in suppressing the non-specific adhesion of T lymphocytes. The characteristic group-containing hydrophilic hydrocarbon chains which the spacer is allowed to have as its skeleton structure include the same alkyl chains as mentioned above, excepting they contain a hydroxyl group, a carboxyl group, or such a dissociable functional group as amino group. They may contain a carbon double bond, a cyclic substituent, or a branch. Among other hydrophilic hydrocarbon chains mentioned above, ethylene oxide skeletons having a polymerization degree in the range of 1 to 50, preferably 4 to 30, prove to be more preferably in the sense that they permit the spacer to manifest a notably high effect in suppressing the non-specific adhesion of T lymphocytes.

As preferred examples of the compound which forms covalent bonds each with the surface of the water-insoluble solid substance and the ligand and serves effectively as the spacer in the B lymphocyte separating material of this invention, the compounds possessing the following structures may be cited. Of course, there are not the only compounds available for the purpose.

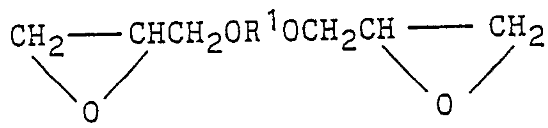

(I)

wherein $R^1$ is a linear alkyl having a number of carbon atoms in the range of 2 to 20, preferably having a number of repeating methylenes in the range of 4 to 10.

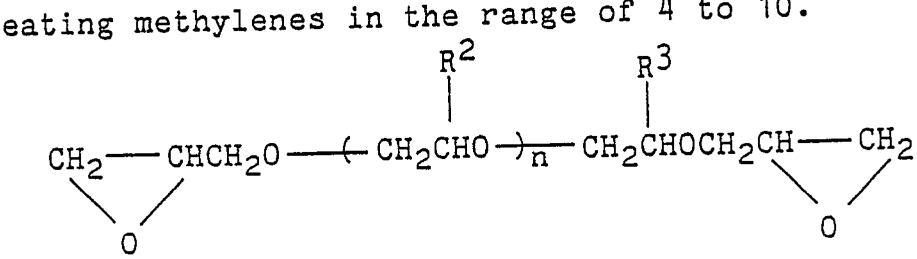

(II)

wherein $R^2$ and $R^3$ independently are hydrogen atom or an alkyl group of 1 to 4 carbon atoms and n is a number in the range of 0 to 49, preferably 3 to 29.

The production of the B lymphocyte separating material of the latter type of this invention is accomplished, for example, by selecting a compound such as a bisepoxide represented by the structural formula (I) or (II) mentioned above which possesses reactive functional groups one each at the opposite terminals thereof and also possesses a molecular skeleton of a chain length suitable for use as a spacer and causing the reactive functional groups of this compound to react respectively with the surface of the water-insoluble solid substance and the amino group or carboxyl group of the ligand so as to form covalent bonds therewith by any of the known methods employed in the field of immobilized enzymes or affinity chromatography. Consequently, the ligand possessing high affinity for the immunoglobulin is strongly joined by the covalent bond to the surface of the water-insoluble solid substance through the medium of the spacer.

B LYMPHOCYTE SEPARATING METHOD AND SEPARATING DEVICE

The B lymphocyte separating method of the present invention effects efficient separation of B lymphocyte from a B lymphocyte-containing liquid by allowing the B lymphocyte-containing liquid to contact the aforementioned separating material having fixed on the surface of a water-insoluble solid substance an organically synthesized ligand possessing high affinity for the immunoglobulin G or the separating material having fixed on the surface of a water-insoluble solid substance through the medium of a spacer an organically synthesized ligand possessing high affinity for the immunoglobulin thereby inducing selective sequestration of the B lymphocytes possessing the immunoglobulin molecules (S-Ig) on the surface of membrane.

The B lymphocyte-containing liquids on which the B lymphocyte separating method of this invention is

effectively carried out by way of treatment include whole blood, the leukocyte fraction obtained by subjecting the whole blood to a centrifugal treatment, and the lymphocyte suspension, for example. The lymphocyte suspension is prepared by treating a lymphocyte-containing cell suspension such as the peripheral blood or the tissue liquid with a blood plasma separator or a centrifugal separation device thereby obtaining the lymphocyte fraction in a refined form from the other blood cell components, and susperding the lymphocyte fraction in a suitable solution. The solution for suspending the lymphocyte fraction may be a simple saline or a solution containing the serum of the subjects own, for example.

The B lymphocyte separating method of this invention is capable of conveniently and quickly separating and removing the B lymphocytes in high yield from the blood and inflicts functional damage only slightly on the blood components during the course of the separation. Thus, it can be utilized for the diagnosis and therapy of diseases concerning the immune function and for the basic technique intended for the acquisition of a physiologically active substance originating in lymphocytes. For the purpose of therapy, in the diseases indicated by abnormal propagation of B cells such as primary immune failure syndromes represented by sex-linked lymphocytosis syndromes or lymphatic tumors represented by pre-B cellular lymphatic leukemia, B cellular malignant lymphatic leukemia, follicular lymphoma, Burkitt lymphoma, macroglobulinemia, μ chain disease, myeloma, γ chain disease, and α chain disease, it is considered that when the abnormally propagated B cells are effectively removed, the symptoms are alleviated because the ratios of lymphocyte subclass and the subpopulation are changed and the signals from the T cells for supressing abnormal propagation and abnormal differentiation (suppressor T cells for suppresion of

propagation and helper T cells for promotion of differentiation) begin to function effectively.

In the B lymphocyte separation method of the present invention, the contact of the B lymphocyte separating material with the B lymphocyte-containing liquid such as the lymphocyte suspension may be attained by causing the B lymphocyte-containing liquid to contact the B lymphocyte separating material in the form of a flat plate. For the purpose of enabling the surface of the water-insoluble solid substance to maintain the organically synthesized ligand in the absolutely necessary amount and offer an ample space for adorption, however, the contact is advantageously accomplished by employing a B lymphocyte separating device provided with a column which is packed with the B lymphocyte separating material in a particulate form and passing the B lymphocyte-containing liquid through this B lymphocyte separating device. In the B lymphocyte separating device provided with a column which is packed with the B lymphocyte separating material in a particulate form, the materials which are usable in forming the container of the column include synthetic resins such as polypropylene, polycarbonate, polystyrene, and polymethyl methacrylate, glass, and metals such as stainless steel. Among other materials mentioned above, polypropylene and polycarbonate prove to be particularly desirable because they tolerate the impact of sterilization in an autoclave and allow ease of handling. The column of the B lymphocyte separating device is preferable to be provided between the outlet/inlet thereof and the separating material bed packed with the B lymphocyte separating material with a filter possessing meshes pervious to the cell component in the B lymphocyte-containing liquid and impervious to the separating material. The material to be used in forming the filter is required to be physiologically inert and rich in strength. This material is desired to be polyester or polyamide.

Fig. 1 is a model diagram illustrating the manner in which a typical B lymphocyte separating device as one embodiment of this invention is used. As illustrated in Fig. 1, a lymphocyte suspension 1 as a B lymphocyte-containing liquid is introduced by a pump 2 through an inlet 9 of the B lymphocyte separating device into a column 6 packed with a B lymphocyte separating material 5. The B lymphocyte separating material is retained in place inside the column 6 by means of filters 4a, 4b. After the introduction of the lymphocyte suspension 1, a washing liquid 8 is injected by a pump 3 into the column 6 to rinse the interior of the column for expulsion of the cells which have escaped being sequestered and have been remaining inside the column. A treated liquid 7 flowing out of an outlet 10 is recovered. The treated liquid 7 constitutes itself a fraction not adsorbed by the separating material and contains T lymphocytes with high purity in high yield.

As the washing liquid 8 to be used for washing off the unsequestered cells (T lymphocytes) without inducing the elution of the cells (B lymphocytes) sequested by the B lymphocyte separating material 5, an isotonic buffer solution or a cell culture liquid may be used. It is preferable to use an isotonic buffer solution free from a divalent cation, preferably Hanks balanced salt solution (HBSS). The passage of the lymphocyte suspension 1 may be carried out continuously or incontinuously, as occasion demands. It may incorporate therein a prescribed length of incubation.

The recovery of the cells which have been sequestered by the B lymphocyte separating material 5 may be effected by removing the unsequestered cells with the washing liquid 8 and then weakening by suitable means the binding force between the cells sequestered on the B lymphocytes separating material 5 and the surface of the B lymphocyte separating material 5. To be specific, this recovery is accomplished by using an isotonic buffer

solution containing an amino acid, saccharides, or albumin as an eluant and causing the flow of the eluant by exerting physical vibration on the column 6 thereby desorbing and recovering the sequestered cells.

In the B lymphocyte separating method of the present invention, for the purpose of lowering the non-specific adhesiveness of the T lymphocytes relative to the B lymphocyte separating material and improving the yield, it is allowable to treat the separating material as with bovine serum albumin before it is put to use in the operation of B lymphocyte separation as widely practised in the art.

BODY FLUID PURIFYING MATERIAL

The body fluid purifying material of this invention is characterized by having a dipeptide or a derivative thereof fixed on the surface of a water-insoluble carrier.

The dipeptide or derivative thereof which is used as a ligand in the body fluid purifying material of this invention is a compound of two identical or different amino acids having the carboxyl group of one of the amino acids linked by an acid amide bond, namely a peptide bond, to the amino group of the other amino acid in consequence of dehydration or a derivative of the compound. The dipeptides or derivatives thereof which answer the description include dipeptides of acidic amino acids such as aspartic acid, glutamic acid, asparagine, and glutamine with aromatic ring amino acids such as phenylalanine, tyrosine, diiodotyrosine, and surinamine or with heterocyclic amino acids such as tryptophan, proline, hydroxyproline, and histidine or derivatives of the dipeptides. Among other dipeptides or derivatives mentioned above, the dipeptides of aspartic acid or glutamic acid with phenylalanine, tyrosine, tryptophan, proline, and hydroxyproline or derivatives of the peptides prove to be suitable. Particularly desirable of these dipeptides or derivatives thereof are aspartylphenylalanines, i.e. dipeptides of aspartic acid and

phenylalanines, or derivatives thereof. Especially, aspartylphenylalanine methyl ester is suitable.

In the interaction of this dipeptide with a pathogenic substance, particularly an immunoglobulin type compound, the hydrophobicity of the principal part of the compound and the electrostatic characteristic due to the hetero atom manifest their respective interactive forces with the amino acid residue at the adsorption site in the molecular chain of the pathogenic substance. The interactive force coupled with the fact that the steric structure of the compound in question suitably fits into the intramolecular pocket of the pathogenic substance is thought to give rise to the high affinity for the non-antigen/antibody system.

The water-insoluble carrier which are usable herein for fixing the organically synthesized ligand in the body fluid purifying material of this invention include organic macromolecular compounds of agarose type, dextran type, cellulose type, polyacrylamide type, polyvinyl alcohol type, polyvinyl pyrrolidone type, polyacrylonitrile type, styrene-divinylbenzene copolymer type, polystyrene type, acrylic ester type, methacrylic ester type, polyethylene type, polypropylene type, polyethylene 4-fluoride type, ethylene-vinyl acetate copolymer type, polyamide type, polycarbonate type, polyvinylidene fluoride type, polyvinylformal type, polyarylate type, and polyether sulfone type, inorganic substances of glass type, alumina type, titanium type, activated carbon type, and ceramic type, and collagen and chitosan which are natural organic macromolecular substances of biotic origin. The known carriers which are used in immobilized enzymes and affinity chromatography can be used without any specific restriction. The shape in which the water-insoluble carrier is to be used in the present invention is not specifically defined. The water-insoluble carrier can be used in any of various forms such as, for example, particles, fibers, hollow fibers, membranes, and

flat plates. In terms of the perviousness to such body fluids as blood and blood plasma and the convenience of handling during the preparation of the purifying material, the water-insoluble carrier is desired to be in the form of particles having an average diameter in the range of 0.05 to 5 mm. The average particle diameter as mentioned herein is what is determined by classifying a given sample of particles with sieves defined in Japanese Industrial Standard (JIS) Z-8801, registering the intermediate value between the upper limit and the lower limit of particle diameters in each of the classes, and averaging the weights of these intermediates representing the relevant classes. The individual particles of the water-insoluble carrier are desired to be in a spherical shape in the sense that such particles do not readily inflict physical damage to the cells and they are produced easily with high uniformity of size. The water-insoluble carrier, for the purpose of retaining as large an amount of the dipeptide or a derivative thereof as permissible on the surface thereof and manifesting as high adsorption efficiency as possible, is desired to possess a porous structure. The individual pores of the porous structure are preferable to have an average diameter in the range of 100 to 5,000 Å, more preferably 200 to 3,000. When the water-insoluble carrier has a porous texture, the reason for setting the average diameter of the pores in the range of 100 to 5,000 Å is that the diffusion of the pathogenic substances in the body fluid into the pores is inhibited possibly to an extent of decreasing the amount of the pathogenic substances to be adsorbed if the average diameter is smaller than 100 Å, whereas the porous carrier possesses insufficient strength and insufficient surface area and consequently fails to withstand the impact of normal use if the average diameter exceeds 5,000 Å. The term "average particle diameter" as used herein refers to the numerical value to be registered by a mercury pressure porosimeter which is adapted to determine the diameter of

pores in a porous sample by forcing mercury to enter the pores in the sample under pressure, finding the total volume of pores on the basis of the amount of mercury occupying the pores, and computing the pore diameter on the basis of the magnitude of pressure required for the entry of mercury in the pores.

In due consideration of all of these points, porous acrylic ester type particles and porous chitosan particles may be cited as particularly desirable examples of the water-insoluble carrier.

The fixation of the dipeptide or a derivative thereof on the surface of the water-insoluble carrier in the body fluid purifying material of this invention may be accomplished by any of all of the known methods such as those resorting to covalent bond, ion bond, physical adsorption, wrapping and precipitative insolubilization on the surface of carrier. In view of the ability of the fixed ligand to dissolve out, it is desirable to have the dipeptide or a derivative thereof fixed by virtue of covalent bond and then insolubilize it in situ prior to use. For this purpose, the method of activating the insoluble carrier and the method of fixing the ligand generally practised in the field of immobilized enzymes and affinity chromatography are used advantageously herein. It is further permissible, when occasion demands, to have a spacer of desired length interposed between the surface of the water-insoluble carrier and the dipeptide or a derivative thereof serving as the ligand. The space to be used in this case is required only to assume a molecular form and to be capable of being interposed in a desired length between the surface of the water-insoluble carrier and the ligand. It may be assume a skeleton structure of any sort so long as the skeleton structure refrains from causing steric hindrance of the bond between the surface of the water-insoluble carrier and the ligand. When the spacer is interposed as described above between the surface of the

water-insoluble carrier and the ligand, the flowability and the volume-expelling effect of the spacer inhibit the body fluid component from approaching the surface of the purifying material and inducing an interaction therewith, with the possible result that the adhesion of the albumin fraction and others which have a weak interacting force with the purifying material is suppressed preferentially. Consequently, there is produced an effect of curbing the non-specific adhesion of non-pathogenic substances. Even when the spacer is interposed between the surface of the water-insoluble carrier and the ligand, the fixation thereof may be accomplished by any of the various known methods mentioned above. Preferably, however, the fixation by virtue of covalent bond is used.

BODY FLUID PURIFYING DEVICE

The body fluid purifying device of this invention is characterized by being provided with a column packed with a body fluid purifying material having a dipeptide or a derivative thereof fixed on the surface of a water-insoluble carrier of the kind described above, preferably a water-insoluble carrier in a particulate form.

In the body fluid purifying device provided with the column packed with the body fluid purifying device, the materials which are usable for the formation of the container of the column include synthetic resins such as polypropylene, polycarbonate, polystyrene,and polymethyl methacrylate, glass, and metals such as stainless steel. Among other materials mentioned above, polypropylene and polycarbonate prove to be particularly desirable because they tolerate the impact of sterilization in an autoclave and allow ease of handling. The column of the B lymphocyte separating device is preferable to be provided between the outlet/inlet thereof and the body fluid purifying material bed packed with the body fluid purifying material with a filter possessing meshes pervious to the cell component in the B lymphocyte-containing liquid and impervious to the

separating material. The material to be used in forming the filter is required to be physiological inert and rich in strength. This material is desired to be polyester or polyamide.

Fig. 2 is a cross section illustrating in model a typical body fluid purifying device as one embodiment of the present invention. In this working example, a body fluid purifying device 11 has a column container 14 provided with a fluid inlet 12 and a fluid outlet 13 and packed with a body fluid purifying material 15 in a particulate form. The purifying material 15 is retained fast inside the column container 14 by means of filters 6a, 6b disposed near the fluid inlet 12 and the fluid outlet 13.

The therapy of extracorporeal circulation of blood by the use of this body fluid purifying device 11 is carried out with the body fluid purifying device 11 incorporated in a circuit configured as illustrated in Fig. 3. The blood from a patient is introduced through a blood inlet 17 into the circuit, then supplied at a fixed flow rate through a blood pump 18 and a chamber 19 into a blood plasma separating device 20, therein to be separated into the blood component and the blood plasma component. The blood plasma released through a blood plasma outlet 21 of the blood plasma separating device 20 is forwarded through a blood plasma pump 22 and a chamber 23 and introduced through the fluid inlet 12 into the body fluid purifying device 11, therein to be subjected to a treatment of adsorption with the body fluid purifying material 15 contained in the column container 14, and then released through the fluid outlet 13. In a mixing chamber 24, the blood plasma component which has undergone the treatment inside the body fluid prefying device 11 is mixed with the blood component 1 released through a blood outlet 25 of the blood plasma separating devcie 20. The resultant mixture is forwarded through a constant temperature bath 26 and returned into the patientś body through a blood outlet 27.

The body fluid purifying device of this invention, prior to use, is given a wet-hot sterilizing treatment by filling the device with purified water or physiological saline solution, for example, and then heating the device externally.

METHOD FOR AUTOMATIC BLOOD PLASMA PURIFICATION

The method for automatic blood plasma purification according with this invention comprises separating the blood led out of a patient's body into the blood component and the blood plasma component, causing the blood plasma component to contact the body fluid purifying material having the dipeptide or a derivative thereof fixed on the surface of the water-insoluble carrier of the type described above, causing the blood plasma component resulting from the treatment with the body fluid purifying material to be mixed with the blood component, and returning the resultant mixture to the patient's body.

The body fluid purifying material having the dipeptide or a derivative thereof fixed on the surface of the water-insoluble carrier as described above is capable of selectively adsorbing the pathogenic substances contained in the body fluid, namely harmful proteins contained in the body fluid, including autoantibodies such as ordinary immunoglobulins (IgA, IgD, IgE, IgG, and IgM), rheumatoid factors, antinucleic antibody, anti-DNA antibody, anti-lymphocyte antibody, anti-red blood cell antibody, anti-platelet antibody, anti-acetylcholine receptor antibody, anti-colon antibody, antiglomerulus body, pulmonary basement membrane antibody, antiderman spinal intercellular desmosome antibody, anti-insulin receptor antibody, anti-myelin antibody, and anti-hemophilia VIII factor antibody, reduction products of immunoglobulin, complexes between immunoglobulins or between immunoglobulin and other substance (particularly an antigen or an antigen-like substance), lymphokine, complements, and fibrinogen,

particularly immunoglobulin type compounds, and on the other hand, is incapable of exhibiting any adsorbing property to albumin and other useful blood plasma components. When this body fluid purifying material is used in the method for automatic blood plasma purification, it can be expected to produce a literally conspicuous therapeutic effect in such diseases as autoimmune diseases represented by myasthenia gravis, articular rheumatism, and lupus erythematosus, immunity-associated diseases represented by glomerulonephritis, bronchial asthma, and multiple neuritis, rejection symptoms attendant upon transplantation of internal organs, liver failure, hypertension, and cancerous disease which are caused by the aforementioned pathogenic substances without entailing such secondary effects as abnormal change of osmotic pressure peculiar to the hypoproteinemia and consequent edema.

Though the method of this invention for automatic blood plasma purification may be performed batchwise, it is desirable from the standpoint of alleviation of the patients burden to carry out this method continuously with the device incorporated in the extracorporeal blood circulation circuit as described in the preceding paragraph dealing with the body fluid purifying device.

EXAMPLES

Now, the present invention will be described more specifically below with reference to working examples.

Example 1

A B lymphocyte selective sequestration type separating material was prepared by using oxirane group-containing acryl beads [produced by Röhm Pharma and marketed under trademark designation of "Eupergit C" (EC)] (having an average particle diameter of 0.15 mm) as a water-insoluble solid substance and having sulfathiazole (ST), a heterocyclic compound, fixed as a ligand on the solid substance.

First, sulfathiazole was dissolved in an amount calculated to account for a concentration of 0.1 M in 100 ml of a mixture of a 0.2-M carbonate buffer (pH 10) and dimethyl formamide (2 :: 3 in volume ratio). In the resultant solution, the oxirane-acryl beads (having a crosslinking monomer content of 30% by weight) added thereto in a ratio of 0.1 to 0.2 mg/ml were deaerated. The deaerated beads were heated in a water bath at 80°C for three hours and then stirred in a blood mixer (produced by Kayagaki Irika Kogyo K.K. and marketed under product code of "BM-101") overnight at normal room temperature. The beads, with 1 M (pH 8) of monoethanolamine solution added thereto, were stirred overnight for the removal of unaltered oxirane group. Then, the beads were washed sequentially with distilled water, a 0.02-M (pH 4 acetate buffer solution containing 0.5 M of sodium chloride, and a 0.2-M (pH 10) carbonate buffer solution, to obtain sulfathiazole-fixed acryl beads (EC-ST).

A column formed of a Teflon tube 3 mm in inside diameter and 4 mm in outside diameter was packed with the separating material obtained as described above and terminals fitted with a polyester filter (150 mesh) were connected one each to the opposite ends of the tube, to retain the adsorbent inside the column. Hanks' balanced salt solution (HBSS; pH 7.2, free from $Ca^{2+}$ and $Mg^{2+}$) was used to displace the extraneous fluid entrapped inside the column packed with the adsorbent. The column was then put to use in the subsequent operation for the separation of T and B lymphocytes.

Separately, a lymphocyte suspension was prepared by isolating a lymphocyte fraction from the mesenteric lymph node of Wister rat and suspending and diluting this lymphocyte fraction in HBSS in a concentration of 800 x $10^4$ cells/ml. This lymphocyte suspension was stored as ice cooled and used within one hour of the dilution.

By the use of a disposable syringe and a syringe pump (both made by Terumo K.K.), 1 ml of the lymphocyte suspension prepared as described above was injected into the column packed with the separating material and immediately the HBBS was similarly injected at a flow rate of 2 ml/min. into the column to wash the column interior and complete the operation of the separation. The treated liquid consequently recovered was tested for cell density by the use of an automatic blood cell counting device (produced by Ortho Instruments and marketed under product code of "ELT-8"). Then, the B lymphocytes alone in the treated liquid were subjected to fluorescent staining withthe FITC having bonded thereto the antibody against the rat IgG (produced by Miles Corp.) and were counted with the aid of a fluorescent microscope, for calculation of the ratio of T and B lymphocytes in the treated liquid. In consequence of the operation described above, the yield (y) and the purity (p) of the T lymphocytes in the treated liquid and the sequestrability (SR) as the index of the specificity to B lymphocytes were obtained as shown in Table 1.

Example 2

Acryl beads having sulfathiazole, arginine, aspartic acid, phenylalanine, and proline fixed thereon (EC-STA) were prepared by following the procedure of Example 1, except that oxirane group-containing acryl beads [produced by Röhm Pharma and marketed under trademark designation of "Eupergit C"] were used as a water-soluble solid substance and sulfathiazole and a mixed liquid prepared by mixing arginine, aspartic acid, phenylalanine, and proline at a ratio of 16 : 1 : 1 : 1 : 1 were used instead.

The operation for the separation of T and B lymphocytes was carried out in the same manner as in Example 1, using the EC-STA. The results obtained of the yield (y), the purity (p), and the sequestrability (SR) were as shown in Table 1.

Example 3

A B lymphocyte selective sequestration type separating material having sulfathiazole fixed thereon was prepared by using cross-link type chitosan beads (produced by Fuji Spinning Co., Ltd. amd marketed under trademark designation of "Chitopearl (CH)") as a water-insoluble solid substance.

First, the chitosan beads were immersed and deaerated in an aqueous 5 V/v% glutaraldehyde (GA) solution adjusted in advance to pH 7.0 with sodium hydroxide. The deaerated beads were stirred in the blood mixer (produced by Kayagaki Irika Kogyo K.K. and marketed under product code of "BM-101") overnight at normal room temperature. The reaction product thus obtained was washed with distilled water, deaerated in a 0.1-M sulfathiazole solution in a mixed liquid of dimethylformamide and an aqueous 10-mM calcium chloride solution (pH 8) (2 : 3 in volume ratio), stirred overnight in the blood mixer, and then washed with dimethylformamide and distilled water to clean the chitosan beads. Then, the cleaned beads were deaerated in a 1-M ethanolamine (pH 8) solution to effect the blocking of the unaltered aldehyde group and were subsequently stirred overnight in the blood mixer. The beads were again washed with distilled water, placed in an aqueous 10-mM calcium chloride solution (pH 8) containing 1 w/v% of sodium borohydrade and left reacting therein 4°C, to reduce the azomethine group produced by the reaction of the aldehyde group of glutaraldehyde and chitosan and the amino group of sulfathiazole and stabilize the reaction product. Finally, the stabilized beads were washed several times with distilled water, a 0.02-M acetate buffer solution (pH 4) containing 0.5 M of sodium chloride, and a 0.2-M carbonate buffer solution (pH 10), to obtain sulfathiazole-fixed chitosan beads (CH-GAST).

The operation for the separation of T and B lymphocytes was carried out in the same manner as in Example 1, using the CH-GAST. The results obtained of the yield

(y), the purity (p), and the sequestrability (SR) were as shown in Table 1.

Control 1

The operation for the separation of T and B lymphocytes was carried out by following the procedure of example 1, except that acryl beads (EC) having no bonded ligand was used instead for comparison. The results were as shown in Table 1.

Control 2

The operation for the separation of T and B lymphocytes was carried out by following the procedure of example 1, except that chitosan beads (CH) having no bonded ligand was used instead for comparison. The results were as shown in Table 1.

It is clearly noted from the results that the introduction of a ligand possessing high affinity for immunoglobulin brought about a remarkable improvement of the sequestrability of B lymphocytes and consequently heightened the T lymphocyte concentration in the recovered cell suspension.

Example 4

A column was packed with the same sulfathiazole-fixed acryl beads as obtained in Example 1. Then, a 0.5% bovine serum albumin was injected into the column and left standing therein for 16 hours. The free albumin which consequently occurred inside the column was released. By using the sulfathiazole-fixed acryl beads (AEC-ST) thus treated with the bovine serum albumin, the operation of the separation of T and B lymphocytes was carried out in accordance with the procedure of Example 1. The results obtained of the yield (y), the purity (p), and the sequestrability (SR) were as shown in Table 1.

It is clearly noted from the results of Table 1 that the treatment with the albumin was observed to produce an improvement of not less than 20% in yield of the efficiency of separation as compared with the EC-ST (Example 1) which

was given no albumin treatment, without any sacrifice of the purity. This improvement may be explained by a supposition that the adsorbed albumin manifested a surfactant-like effect of supressing the non-specific adhesiveness of T lymphocytes without lowering the affinity between the B lymphocytes and the ligand.

The yield (y), the purity (p), and the sequestrability (SR) were calculated as follows.

$$\text{Yield (y)} = \frac{\text{Number of T lymphocytes in recovered treated liquid after passage through the column}}{\text{Number of T lymphocytes in injected lymphocyte suspension before passage through the column}} \times 100$$

$$\text{Purity (p)} = \frac{\text{Number of T lymphocytes in recovered treated liquid}}{\text{Number of lymphocytes in recovered treated liquid}} \times 100$$

$$\text{Sequestrability (SR)} = \frac{\text{Ratio of adsorption of B lymphocytes to separating material possessing ligand}}{\text{Ratio of adsorption of a lymphocytes to separating material possessing no ligand}}$$

Table 1

| | Separating material | Flow rate (ml/min) | | | | | | | | |
|---|---|---|---|---|---|---|---|---|---|---|
| | | 1.0 | | | 2.0 | | | 3.0 | | |
| | | P(%) | Y(%) | SR | P(%) | Y(%) | SR | P(%) | Y(%) | SR |
| Control 1 | EC | 75 | 60 | 1.0 | 70 | 70 | 1.0 | 70 | 75 | 1.0 |
| Example 1 | EC-ST | 80 | 55 | 1.1 | 80 | 75 | 1.2 | 75 | 75 | 1.2 |
| Example 2 | EC-STA | 90 | 40 | 1.4 | 85 | 70 | 1.6 | 80 | 70 | 1.4 |
| Example 4 | AEC STA | 80 | 95 | 1.0 | 80 | 100 | 1.1 | - | - | - |
| Control 2 | CH | 70 | 90 | 1.0 | 65 | 100 | * | 65 | 100 | * |
| Example 3 | CH-GAST | 85 | 75 | 4.0 | 80 | 75 | * | 80 | 80 | * |

* In the case of CH (Control 2), no calculation was allowed because virtually no B lymphocyte was obserbed to be sequestered within the column.

The T and B lymphocyte separating materials conforming to the present invention (Examples 1 to 4) could treat at least about 1.0 x 10 to 6.0 x 10 lymphocytes per g of separating material in a matter of about 2 minutes/column and, even from the standpoint of vitality and form of adhesion, were not inferred to have inflicted any noticeable damage on the cells.

Example 5

Sulfathiazole was bonded to and fixed on the cross-link type chitosan beads through the medium of glutaraldehyde to obtain sulfathiazole-fixed chitosan beads (CH-GAST) by following the procedure of Example 3, except that an aqueous 5 w/v% glutaraldehyde solution prepared by diluting to 10 times the original volume with a phosphate buffered physiological saline solution of pH 7.4 was used instead.

A column formed of a Teflon tube 3 mm in inside diameter and 4 mm in outside diameter was packed with the separating material obtained as described above and terminals provided with a polyester filter (150 mesh) were connected one each to the opposite terminals of the tube, to retain the separating material fast in the column. Hanks' balanced salt solution (HBSS; pH 7.2, free of Ga and Mg ) was used to displace the extraneous fluid entrapped inside the column packed with the separating material. The column was then put to use in the subsequent operation for the separation of T and B lymphocytes.

Separately, the lymphocytes to be subjected to the operation were collected from human peripheral blood by specific gravity centrifugal separation using Ficoll-paque (specific gravity 1.077, produced by Pharmacia) and then suspended and diluted in the HBBS in a concentration of 800 x 10 cells/ml, to prepare lymphocyte suspension.

By the use of a disposable syringe and a syringe pump (both made by Terumo K.K.), 1 ml of the lymphocyte suspension prepared as described above was injected at a fixed flow rate shown in Table 2 into the aforementioned column packed with the separating material. Immediately, the HBBS was similarly injected at a fixed flow rate shown in Table 2 to wash the interior of the column and complete the operation of the separation. The number of cells in the recovered lymphocyte suspension was measured by the use of an automatic blood cell counting device (produced by Ortho Instruments and marketed under product code of "ELT-8"). Then, the B lymphocytes alone in the recovered liquid were subjected to fluorescent staining with the FITC having bonded thereto an antibody against human immunoglobulin (produced by Miles Corp) and the FITC-positive cells in the recovered liquid were counted with the aid of a fluorescent microscope, to find the ratio of FITC-stained cells (FITC (+)) to the FITC-unstained cells (FITC (-)) sequestered in the column. The results were as shown in Table 2.

Example 6

A B lymphocyte separating material was produced by causing α-L-aspartylphenylalanine methyl ester (AT; produced by Ajinomoto Co, Ltd.), a dipeptide formed of aspartic acid with phenylalanine, to be bonded and fixed as a ligand on cross-link type chitosan beads as a water-insoluble carrier. This B lymphocyte separating material was labeled as CH-GAAT.

First, the chitosan beads were placed and deaerated in an aqueous 5 V/v% glutaraldehyde solution prepared by diluting to 10 times the original volume with a phosphate

buffered physiological saline (PBS) of pH 7.4. Then, the deaerated beads were stirred in the blood mixer (produced by Kayagaki Irika Kogyo K.K. and marketed under product code of "BM-101") at normal room temperature. The resultant reaction product was washed with distilled water was placed and deaerated in an aqueous 0.1 M α-L-aspartylphenylalanine methyl ester solution prepared by dissolving α-L-aspartylphenylalanine methyl ester in a phosphate buffered physiological saline (PBS) of pH 7.4. The deaerated reaction product was stirred overnight in the blood mixer.

Then, the chitosan beads were washed, deaerated in an aqueous 1 M ethanolamine (pH 8) solution to effect the blocking of the unaltered aldehyde group, and stirred overnight in the blood mixer. The beads were washed again with distilled water, placed in an aqueous 10 mM calcium chloride solution containing 1 w/v% of sodium borohydrade and left reacting therein at 4°C to reduce the azomethine group formed in consequence of the reaction of the aldehyde group of glutaraldehyde with chitosan and the amino group of α-L-aspartylphenylalanine methyl ester and stabilize the reaction product. Finally, the beads were washed several times with distilled water, a 0.02 M acetate buffer solution (pH 4) containing an aqueous 0.5 M sodium chloride solution, and a 0.2 M carbonate buffer solution (pH 10) to obtain chitosan beads having α-L-aspartylphenylalanine methyl ester fixed thereon through the medium of glutaraldehyde (CH-GAAT).

The operation for the T and B lymphocyte separation was carried out by following the procedure of Example 5, using the separating material CH-GAAT obtained as described. The results were as shown in Table 2.

Examples 7 and 8

B lymphocyte separating material were produced by using cross-link type chitosan beads (CH) as a water-insoluble carrier and causing sulfathiazole (ST) and α-L-aspartylphenylalanine methyl ester (AT) severally bonded and

fixed as a ligand on the chitosan beads through the medium of epichlorohydrin (EH). These B lymphocyte separating materials were labeled as CH-EHST (Example 7) and CH-EHAT (Example 8).

First, 2-M NaOH and epichlorohydrin were mixed in a ratio of 4 : 1 and diluted with distilled water to 5 times the original volume. In the resultant solution, the chitosan beads were placed and stirred at 40o to 45oC for two hours by the use of the blood mixer. The resultant reaction product was washed with distilled water, placed in a ratio of 0.1 to 0.7 g/ml one of the two aliquots thereof in a solution of 0.1 M of sulfathiazole in a mixed liquid of DMF and an aqueous 10 mM calcium chloride solution (2 : 3) and the other aliquot in a solution of 0.1 M of α-L-aspartylphenylalanine methyl ester in PBS (pH 7.4) to be deaerated therein, and stirred overnight at normal room temperature in the blood mixer.

The reaction product, in the presence of an aqueous 1 M ethanolamine solution (pH 8) incorporated therein for the purpose of effecting the blocking of the unaltered oxylan group, was stirred overnight. It was then washed sequentially with distilled water, an acetate buffer solution of a concentration of 0.02 M (pH 4) containing 0.5 M of sodium chloride, and a carbonate buffer solution of a concentration of 0.2 M (pH 10). Consequently, chitosan beads having sulfathiazole fixed thereon (CH-EHST) and chitosan beads having α-L-aspartylphenylalanine methyl ester fixed thereon (CH-EHAT) were obtained. By the use of these separating materials, CH-EHST, and CH-EHAT, the operation for the T and B lymphocyte separation was carried out by following the procedure of Example 5, except that the flow rate of the lymphocyte suspension and the washing speed were changed as shown in Table 2. The results were as shown in Table 2.

Controls 3 and 4

The operation for the T and B lymphocyte separation was carried out by following the procedure of Example 5, except that the cross-link type chitosan beads (CH) in the unmodified form were used as a separating material and the flow rate of the lymphocyte suspension and the washing speed were changed as shown in Table 2. The results were as shown in Table 2.

Table 2

| | Separating material | Flow rate (ml/hr) | | Ratio of sequestration (%) | |
|---|---|---|---|---|---|
| | | Injection | Washing | FITC (+) | FITC (-) |
| Example 5 | CH-GAST | 150 | 120 | 85 | 58 |
| Example 6 | CH-GAAT | 150 | 120 | 64 | 8 |
| Control 3 | CH | 150 | 120 | 37 | 20 |
| Example 7 | CH-EHST | 40 | 120 | 78 | 36 |
| Example 8 | CH-EHAT | 40 | 120 | 77 | 18 |
| Control 4 | CH | 40 | 120 | 61 | 39 |

It is clearly noted from Table 2 that the B lymphocyte separating materials conforming to this invention (Examples 5 to 8) were effective in preferentially sequestering B lymphocytes similarly to those shown in Table 1. Further, the B lymphocyte separating materials of Examples 5 to 8, even from the standpoint of vitality and form of adhesion, were not inferred to have inflicted any noticeable damage on the cells.

Example 9

A B lymphocyte separating material was produced by using a bis-epoxide possessing a polyethylene oxide chain (produced by Nagase Chemical Industry Co., Ltd. and marketed under product code of "EG 22") (ethylene oxide repeating degree 22) as a spacer and allowing sulfathiazole, a heterocyclic compound, to be bonded and fixed as a ligand on oxylan group-containing acryl beads [produced by Rohm Pharma and marketed under trakdemark designation of "Eupergit C (EC)] (average particle diameter 0.15 mm). This B lymphocyte separatng material was labeled as EC-EG22ST.

First, 30 g of the EC washed in advance with distilled water was placed and deaerated in a solution of 0.2 M of 1,3-propane diamine in a carbonate buffer solution (pH 10). It was then left reacting in a water bath at 80°C. It was stirred overnight at normal room temperature in a blood mixer (produced by Kayagaki Irika Kogyo K.K. and marketed under product code of "BM-101") and then washed with distilled water. Then, 18 g (wet weight) of the EC having an amino group incorporated therein in consequence of the treatment mentioned above was diluted with a solution of 10% (w/v) EG 22 in a carbonate buffer solution (pH 10) to a total volume of 100 ml. The resultant mixture was stirred overnight in the blood mixer, left reacting in a water bath at 80°C for one hour, and washed with distilled water. The resultant reaction product was placed in 100 ml of a solution of 0.1 M of sulfathiazole in a mixed liquid of a carbonate buffer solution (pH 10) and dimethyl formamide (2

: 3 in volume ratio), left reacting in a water bath at 80°C for three hours, and stirred overnight in the blood mixer. The reaction product, in the presence of 100 ml of ethanolamine of a concentration of 1 M (pH 8) incorporated therein for the purpose of effecting the blocking of the unaltered oxyrane group, was stirred overnight. Then, it was washed sequentially with distilled water, an acetate buffer solution of a concentration of 0.02 M (pH 4) containing 0.5 M of sodium chloride, and a carbonate buffer solution of a concentration of 0.2 M (pH 10). Consequently, acryl beads having sulfathiazole fixed thereon through the medium of a polyethylene oxide chain (EC-EG22ST) were obtained.

A column fomred of a Teflon tube 3 mm in inside diameter and 4 mm in outside diameter was packed with the separating obtained as described above and terminals provided with a polyester filter (150 mesh) were connected one each to the opposite ends of the tube to retain the separating material in the column. Hanks' balanced salt solution (HBSS; pH 7.2, free of $Ca^{2+}$ and $Mg^{2+}$ ) was used to displace the extraneous fluid entrapped inside the column packed with the separating material. Then, the column was put to use in the operation for the T and S lymphocyte separation.

Separately, the lymphocytes subjected to the separation were collected from human peripheral blood by specific gravity centrifugal separation using a Ficoll-paque (specific gravity 1.077, produced by Pharmacia) and suspended and diluted in the HBBS in a concentration of 800 x 10 cells/ml, to prepare a lymphocyte suspension.

By the use of a disposable syringe and a syringe pump (both made of Terumo K.K.), 1 ml of the lymphocyte suspension prepared as described above was injected at a fixed flow rate indicated in Table 3 into the column packed with the separating material and immediately the HBBS was similarly injected at a fixed flow rate indicated in Table 3 into the column to wash the column interior and complete the

operation of the separation. The number of cells in the recovered lymphocyte suspension was determined with an automatic blood cell counting device (produced by Ortho Instruments and marketed under product code of "ELT-8"). Then, the B lymphocytes alone in the recovered liquid were subjected to fluorescent staining with the FITC having bonded thereto an antibody against the human immunoglobulin (produced by Miles Corp). The FITC-positive cells in the recovered liquid were counted with the aid of a fluorescent microscope, to find the ratio of FITC-stained cells (FITC (+)) and FITC-non-stained cells (FITC(-)) sequested in the column. The results were as shown in Table 3.

Control 5

The operation for T and B lymphocyte separation was carried out by following the procedure of Example 9, except that oxirane group-containing acryl beads [produced by Rhom Pharma and marketed under trademark designation of "Eupergit C (Ec)] were used in the unmodified form as a separating material. The results were as shown in Table 3.

Example 10

A B lymphocyte separating material was produced by using a bis-epoxide possessing a polyethylene oxide chain (produced by Nagase Chemical Industry Co., Ltd. and marketed under product code of "EG22") as a spacer and causing sulfathiazole (ST) bonded and fixed as a ligand on cross-link type chitosan beads [produced by Fuji Spinning Co., Ltd. and marketed under trademark designation of "Chitopearl (CH)"]. This B lymphocyte separating material was labeled as CH-EG22ST.

First, 30 g (wet weight) of the chitosan beads washed in advance with distilled water were diluted with a solution of 10% (w/v) EG22 in a carbonate buffer solution (pH 10) to a total volume of 100 ml, stirred overnight in the blood mixer, left reacting in a water bath at 80°C for one hour, and washed with distilled water. The beads were added to 100 ml of a solution of 0.1 M of sulfathiazole in a

mixed liquid of a carbonate buffer solution (pH 10) and dimethyl formamide (2 : 3 in volume ratio), left reacting in a water bath at 80oC for three hours, and stirred overnight in the blood mixer. The reaction product, in the presence of 100 ml of ethanolamine of a concentration of 1 M (pH 8) incorporated therein for the purpose of effecting the blocking of the unaltered oxirane group, was stirred overnight. Then, it was washed sequentially with distilled water, an acetate buffer solution of a concentration of 0.02 M (pH 4) containing 0.5 M of sodium chloride, and a carbonate buffer solution of a concentration of 0.2 M (pH 10). Consequently, chitosan beads having sulfathiazole fixed thereon (CH-EG22ST) were obtained.

The operation for T and B lymphocyte separation was carried out by following the procedure of Example 9, using the separating material CH-EG22ST obtained as described above. The results were as shown in Table 3.

Control 6

The operation for T and B lymphocyte separation was carried out by following the procedure of Example 9, excepting cross-link type chitosan beads (produced by Fuji Spinning Co., Ltd. and marketed under trademark designation of "Chitopearl (CH)") were used in the unmodified form as a separating material. The results were as shown in Table 3.

Table 3

| | Separating material | Flow rate (ml/hr) Injection | Washing | Ratio of swquestration (%) FITC (+) | FITC (-) |
|---|---|---|---|---|---|
| Example 9 | EC-EG22ST | 20 | 60 | 66 | 6 |
| Control 5 | EC | 20 | 60 | 92 | 86 |
| Example 10 | CH-EG22ST | 20 | 60 | 75 | 7 |
| Control 6 | CH | 20 | 60 | 67 | 52 |

It is clearly noted from Table 3 that the separating materials having sulfathiazole bonded thereto through the medium of a spacer (Examples 9 and 10), under the conditions of separation indicated, notably lowered the adhesiveness of lymphocytes themselves, suppressed the non-specific adhesion of T lymphocytes without impairing the high affinity for B lymphocytes, and at the same time exhibited highly accurate T and B lymphocyte selectivity. The manifestation of this high selectivity was observed invariably when the acryl beads (EC) were used or when the chitosan beads were used as a water-insoluble solid substance. This fact indicates that the T and B lymphocyte selectivity does not depend on the kind of the water-insoluble solid substance.

Examples 11 and 12

Acryl beads having sulfathiazole fixed thereon through the medium of an ethylene oxide chain were produced by following the procedure of Example 9, except that a bis-epoxide (produced by Nagase Chemical Industry Co., Ltd. and marketed under product code of "EG1") possessing an ethylene oxide chain having an ethylene oxide repeating degree of 1 (Example 11) or a bis-epoxide (produced by Nagase Chemical Co., Ltd. and marketed under product code "EG9") (Example 12) possessing a repeating degree of 9 was used instead. The B lymphocyte separating materials thus obtained were labeled as EC-EG1ST (Example 11) and EC-EG9ST (Example 12).

The operation for T and B lymphocyte separation was carried out by repeating the procedure of Example 9, except that the separating materials EC-EG1ST or EC-EG9ST was used instead and the flow rate of the lymphocyte suspension injected and the speed of washing were changed as indicated in Table 4. The results were as shown in Table 4.

Example 13

The same separating material, EC-EG22ST, was prepared as in Example 9. Then, the operation for T and B lymphocyte separation was carried out by repeating the procedure of Example 9, except that the separating material

was used instead and the flow rate of the lymphocyte suspension injected and the speed of washing were changed as indicated in Table 4. The results were as shown in Table 4.

Examples 14 and 15

Acryl beads having sulfathiazole fixed thereon through the medium of a methylene chain were produced by following the procedure of Example 9, except that 1,4-butanediol diglycidyl ether possessing a methylene repeating degree of 4 (produced by Tokyo Kasei K.K. and abbriviated "ME4") or 1,6-hexanediol diglycidyl ether possessing a methylene repeating degree of 6 (produced by Tokyo Kasei K.K. and abbreviated "ME6") was used as a spacer and a solution of 10 (w/v)% ME4 in a mixed liquid of a carbonate buffer solution (pH 10) and dimethyl formamide (9 : 1 in volume ratio) or a solution of 10 (w/v)% ME6 in a mixed liquid of a carbonate buffer solution (pH 10), dimethyl formamide, and distilled water (2 : 2 : 1 in volume ratio) was used as the solution therefor. The B lymphocyte separating materials thus obtained were labeled as EC-ME4ST (Example 14) and EC-ME6ST (Example 15).

The operation for T and B lymphocyte separation was carried out by following the procedure of Example 9, except that the separating material, EC-ME4ST or EC-ME6ST, was used instead and the flow rate of the lymphocyte suspension injected and the speed of washing were changed as indicated in Table 4. The results were as shown in Table 4.

Table 4

| | Separating material | Structure | Flow rate (ml/hr) | | Ratio of sequest-ration (%) | |
|---|---|---|---|---|---|---|
| | | | Injection | Washing | EITC (+) | EITC (-) |
| Example 11 | EC-IG1SI | (EC) $-CH_2OCH_2CHCH_2-NH(CH_2)_3NH-CH_2CHCH_2$ (OH under each CHCH₂)<br>$-O(CH_2CH_2O)_1-CH_2CHCH_2-ST$ (OH under CHCH₂) | 20 | 60 | 91 | 57 |
| Example 12 | EC-IG9SI | (EC) $-CH_2OCH_2CHCH_2-NH(CH_2)_3NH-CH_2CHCH_2$ (OH under each CHCH₂)<br>$-O(CH_2CH_2O)_9-CH_2CHCH_2-ST$ (OH under CHCH₂) | 12 | 60 | 85 | 12 |
| Example 13 | EC-IG22SI | (EC) $-CH_2OCH_2CHCH_2-NH(CH_2)_3NH-CH_2CHCH_2$ (OH under each CHCH₂)<br>$-O(CH_2CH_2O)_{22}-CH_2CHCH_2-ST$ (OH under CHCH₂) | 12 | 60 | 85 | 6 |
| Example 14 | EC-MT4SI | (EC) $-CH_2OCH_2CHCH_2-NH(CH_2)_3NH-CH_2CHCH_2$ (OH under each CHCH₂)<br>$-O(CH_2)_4O-CH_2CHCH_2-ST$ (OH under CHCH₂) | 60 | 120 | 67 | 7 |
| Example 15 | EC-MT6SI | (EC) $-CH_2OCH_2CHCH_2-NH(CH_2)_3NH-CH_2CHCH_2$ (OH under each CHCH₂)<br>$-O(CH_2)_6O-CH_2CHCH_2-ST$ (OH under CHCH₂) | 40 | 120 | 96 | 62 |

It is clearly noted from the results of Table 4 that the separating materials having sulfathiazole bonded thereto through the medium of a spacer suppressed the non-specific adhesion of T lymphocytes without impairing the high affinity for B lymphocytes and maintained highly accurate T and B lymphocyte separating ability and particularly those using EG 9 (Example 12), EG 22 (Example 13), and ME 4 (Example 14) as a spacer exhibited prominent T and B selectivity.

Example 16

Chitosan beads having sulfathiazole fixed thereon through the medium of an ethylene oxide chain were produced by following the procedure of Example 10, except that a bis-epoxide possessing an ethylene oxide chain of an ethylene oxide repeating degree of 1 (produced by Nagase Chemical Industry Co., Ltd. and marketed under product code of "EG1") was used as a spacer and a solution of 10 (w/v)% EG1 in a mixed liquid of a carbonate buffer solution (pH 10) and dimethyl formamide (9 : 1 in volume ratio) was used as the solution therefor. The B lymphocyte separating material thus obtained was lebeled as CH-EG1ST.

The operation for T and B lymphocyte separation was carried out by following the procedure of Example 10, except that the separating material CH-EG1ST was used instead and the flow rate of the lymphocyte suspension injected and the washing speed were varied as indicated in Table 5. The results were as shown in Table 5.

Example 17

Chitosan beads having sulfathiazole fixed thereon through the medium of ethylene oxide chain were produced by following the procedure of Example 10, except that trifunctional tri-epoxide possessing three ethylene oxide chains (produced by Nagase Chemical Industry Co., Ltd. and marketed under product code of "EG3") was used as a spacer instead. The B lymphocyte separating material thus obtained was labeled as CH-EG3ST.

The operation for T and B lympohocyte separation was carried out by following the procedure of Example 10, except that the separating material CH-EG3ST was used instead and the flow rate of the lymphocyte suspension injected and the washing speed were varied as indicated in Table 5. The results were as shown in Table 5.

Example 18

Chitosan beads having sulfathiazole fixed thereon through the medium of a polyethylene oxide chain were produced by following the procedure of Example 10, excepting a bis-epoxide possessing a polyethylene oxide chain of an ethylene oxide repeating degree of 9 was used as a spacer. The B lymphocyte separating material thus obtained was labeled as CH-EG3ST.

The operation for T and B lymphocyte separation was carried out by following the procedure of Example 10, except that the separating material CH-EG3ST was used instead and the flow rate of the lymphocyte suspension injected and the washing speed were varied as indicated in Table 5. The results were as shown in Table 5.

Example 19

The same separating material, CH-EG22ST, was prepared as in Example 10. The operation for T and B lymphocyte separation was carried out by following the procedure of Example 10, except that the separating material CH-EG22ST was used instead and the flow rate of the lymphocyte suspension injected and the washing speed were varied as indicated in Table 5. The results were as shown in Table 5.

Example 20 and 21

Chitosan beads having sulfathiazole fixed thereon through the medium a methylene chain were produced by following the procedure of Example 10, except that 1,4-butanediol diglycidyl ether of a methylene repeat that degree of 4 (produced by Tokyo Kasei K.K. and abbreviated "ME4") (Example 20) or 1,6-hexanediol diglycidyl ether of a

methylene repeating degree of 6 (produced by Tokyo Kasei K.K. and abbreviated ME6") (Example 21) as a spacer and a solution of 10 (w/v)% ME4 in a carbonate buffer solution (pH 10) or a solution of 10 (w/v)% ME6 in a mixed liquid of a carbonate buffer solution (pH 10), dimethyl formamide, and distilled water (2 : 2 : 1 in volume ratio) was used as the solution therefor. The B lymphocyte separating materials thus obtained were labeled as CH-ME4ST (Example 20) and CH-ME6ST (Example 21).

The operation for T and B lymphocyte separation was carried out by following the procedure of Example 10, except that the separating material CH-ME4ST or CH-ME6ST was used instead and the flow rate of the lymphocyte suspension injected and the washing speed were varied as indicated in Table 5. The results were as shown in Table 5.

Table 5

| | Separating material | Structure | Flow rate (ml/hr) | | Ratio of sequestration (%) | |
|---|---|---|---|---|---|---|
| | | | Injection | Washing | EITC (+) | EITC (-) |
| Example 16 | CH-IG1SI | 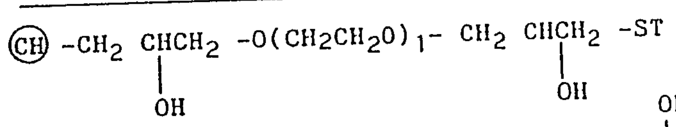 | 40 | 120 | 68 | 14 |
| Example 17 | CH-IG3SI | 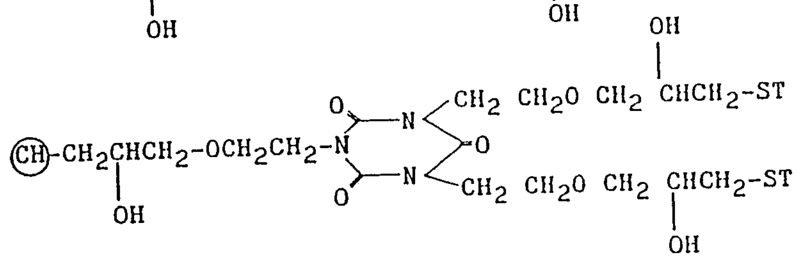 | 40 | 120 | 90 | 21 |
| Example 18 | CH-IG9SI | 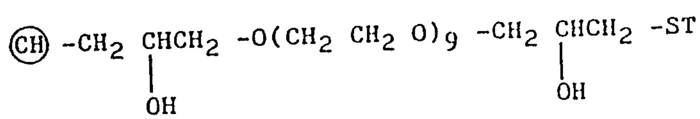 | 15 | 120 | 80 | 20 |
| Example 19 | CH-IG22SI | 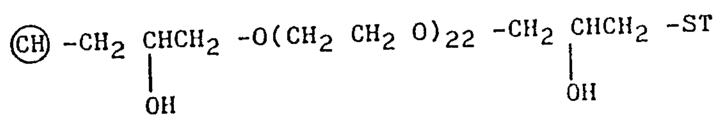 | 12 | 60 | 79 | 6 |
| Example 20 | CH-MT1SI | 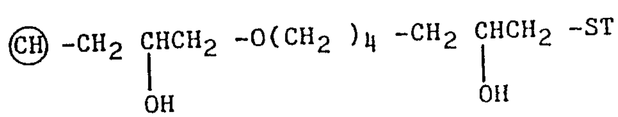 | 40 | 120 | 80 | 4 |
| Example 21 | CH-MT6SI | 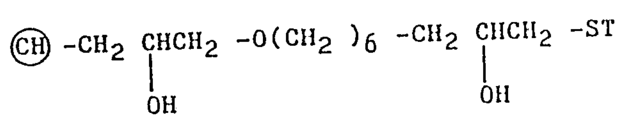 | 40 | 120 | 79 | 8 |

It is clearly noted from the results of Table 5 that the separating materials having sulfathiazole bonded thereto through the medium of a spacer, similarly to those whose results are shown in Table 5, suppressed the non-specific adhesion of T lymphocytes without impairing the high affinity for B lymphocytes and maintained a highly accurate T and B lymphocyte separating ability. Particularly those using EG22 (Example 19), ME4 (Example 20), and ME6 (Example 6) as a spacer exhibited outstanding selectivity as evinced by a meager FITC (-) sequestering ratio of less than 10% and a high FITC (+) sequestering ratio of 80%.

Examples 22 to 25

The lymphocyte fraction obtained from the human peripheral blood by specific gravity centrifugal separation mainly comprises T lymphocytes and B lymphocytes and additionally contains impurities such as monocytes and null cell. The entrainment of these impurites, i.e. the non-T,B lymphocyte type cells (N), with the lymphocyte fraction was evaluated by the peroxidase staining method and the immuno-bead method. To be specific, the operation for T and B lymphocyte separation was carried out by following the procedure of Example 9, except that the EC-EG22ST obtained in the same manner as in Example 9, the CH-EG22ST obtained in the same manner as in Example 10, the CH-ME4ST obtained in the same manner as in Example 20, or the CH-ME6ST obtained in the same manner as in Example 22 was used instead and the flow rate of the lymphocyte suspension injected and the washing speed were changed as indicated in Table 6. The ratio of the non-T,B lymphocyte type cells (N) entrained with the lymphocyte suspension before and after the operation of this separation of this separation was evaluated by the method mentioned above. Further, the yield and the purity of the T lymphocytes recovered by the operation mentioned above were calculated by the method mentioned above, to determine exact separation efficiency. The results were as shown in Table 6.

Table 6

Ratio of cells in lymphocyte fraction injected into the column *1 (%)

To 65~75 | Bo 10~20 | No 5~15

| | Separating material | Flow rate (ml/hr) | | Ratio sequestered in column *2 (%) | | | Ratio of recovered T lymphocytes | |
|---|---|---|---|---|---|---|---|---|
| | | Injection | Washing | T/To | B/Bo | N/No | Purity | Yield |
| Example 22 | TC-FG22ST | 12 | 60 | 20± 3 | 84± 7 | 54± 8 | 89± 2 | 80±12 |
| Example 23 | CH-EG222ST | 12 | 60 | 20±12 | 84±10 | 73± 2 | 89± 4 | 90±13 |
| Example 24 | CH-MF4ST | 40 | 120 | 20± 3 | 89± 4 | 83± 8 | 94± 2 | 82± 5 |
| Example 25 | CH-MF6ST | 40 | 120 | 17± 5 | 90± 3 | 84±15 | 94± 2 | 85± 2 |

*1 - To, Bo, and No are the initial values of T lymphocytes, B lymphocytes, and non-T, B lymphocytes contained in the lymphocyte fraction.

*2 - T/To, B/Bo, and N/No are the values obtained, by dividing, by the initial values To, Bo, and No, the values of sequestered T lymphocytes (T), sequeste red B lymphocytes (B), and sequestered non-T, B lymphocytes (N) calculated reciprocally from the values of T lymphocytes, B lymphocytes, and non-T, B lymphocytes contained in the lymphocyte fraction recovered after the operation of separation respectively.

It is clearly noted from the results of Table 6 that the T, B lymphocyte separating materials conforming to the present invention invariably possessed very high T, B lymphocyte selectivity and showed outstanding levels of yield and purity of T lymphocytes respectively falling in the range of 80 to 95% and in the range of 90 to 95% and further that this outstanding separating ability was not affected by the entrainment of non-T,B type cells.

Examples 26 to 28

CH-EG1ST was produced in the same manner as in Example 16, CH-EG9ST in the same manner as in Example 18, and CH-EG22ST in the same manner as in Example 10.

By the use of these separating materials, the operation for T, B lymphocyte separation was carried out on the lymphocyte suspension prepared from the rat's mesenteric lymph node and the human peripheral blood lymphocyte suspension by following the procedure of Example 9, except that the feed rate of the lymphocyte suspension injected and the washing speed indicated in Table 7 were used instead. For the fluorescent staining of the lymphocytes from the rat, the FITC having bonded thereto an antibody against the rat immunoglobulin G was used. The results were as shown in Table 7.

Table 7

| | Separating material | Flow rate (ml/h) | | Ratio of sequesteration (%) | |
|---|---|---|---|---|---|
| | | Injection | Washing | FITC (+) | FITC (-) |
| Rat | | | | | |
| Example 26 | CH-EG1ST | 40 | 120 | 67 | 15 |
| Example 27 | CH-EG9ST | 40 | 120 | 62 | 16 |
| Example 28 | CH-EG22ST | 40 | 120 | 64 | 13 |
| Human | | | | | |
| Example 26 | CH-EG1ST | 20 | 60 | 86 | 28 |
| Example 27 | CH-EG9ST | 20 | 60 | 63 | 16 |
| Example 28 | CH-EG22ST | 20 | 60 | 69 | 7 |

It is clearly noted from the results of Table 7 that the T, B lymphocyte separating materials conforming to the present invention invariably exhibited very high B lymphocyte affinity for the lymphocytes originating in the rat and the human. From this fact is drawn a fair inference that the T, B lymphocyte separating materials of this invention having fixed thereon through the medium of a spacer a ligand possessing affinity for immunoglobulin G are possessed of B lymphocyte affinity not affected by the species of animal from which the lymphocytes are separated.

Further, the T, B lymphocyte separating materials conforming to the present invention (Examples 9 to 28) were capable of treating approximately at least $1.0 \times 10^7$ to $6.0 \times 10^7$ lymphocytes per g of separating material and, even from the standpoint of vitality and form of adhesion, they were not inferred to have inflicted any noticeable damage on the cells.

Example 29

First, glutaraldehyde was dissolved in a 0.1-M phosphate buffer solution (pH 7.4) in an amount calculated to account for a concentration of 5 (V/v)%. In the glutaraldehyde solution, chitosan beads (Lot No. H-0132, produced by Fuji Spinning Co.,Ltd. and marketed under trademark designation of "Chitopearl BCW3003") were added in a ratio of 0.2 to 0.4 g (wet weight)/ml to be deaerated. The deaerated chitosan beads were stirred overnight at normal room temperature by the use of a blood mixer (produced by Kayagaki Irika Kogyo K.K. and marketed under product code of "BM-101"). Then, the reaction product consequently obtained was washed with distilled water, placed in a solution of 0.05 M of aspartylphenylalanine methyl ester (produced by Ajinomoto Co., Ltd. and marketed under trademark designation of "Pal") in a 0.1-M carbonate buffer solution and deaerated therein, and stirred overnight at normal room temperature by the use of a blood mixer. The reaction product was washed with distilled water and, in the

presence of 1 M of monoethanol amino (pH 8.0) incorporated therein for the purpose of effecting the blocking of the unaltered aldehyde group, deaerated, and stirred overnight in the blood mixer.

The resultant reaction product was washed with distilled water, immersed in a solution of 0.1 M of phosphate buffer solution (pH 7.4) in 1 (w/v)% sodium borohydrade, and left reacting therein with occasional stirring for three hours. In consequence of this reaction, the azomethine bond produced by the reaction of the aldehyde group of glutaraldehyde, and the amino group of chitosan and the aspartylphenylalanine methyl ester was reduced and the reaction product was stabilized. Further by repeatedly washing the reaction product with distilled water, a 0.02-M acetate buffer solution having a pH 4.0 and containing 0.5 M of sodium chloride, and a 0.2-M carbonate buffer solution having a pH 10, to prepare a purifying material. This purifying material was put to use in the following experimental adsorption. First in the experimental adsorption, 1 g (wet weight) of the purifying material obtained as described above and 5 ml of normal human blood plasma incorporating therein 140 ul of a CPD solution as an anticoagulant per ml were placed in a test tube of glass (made by Terumo K.K. and marketed under trademark designation of "Larubo"), deaerated, and stirred by the use of a blood mixer and, at the same time, incubated in a hot air circulation type constant temperature bath (produced by Tabai K.K. and marketed under product code of "P(S)-212 type") at 37oC for 90 minutes. On elapse of a prescribed time, the mass resulting from the incubation was passed through a polyester mesh (225 mesh). The filtrate was tested for albumin content and total protein content respectively by the bromocresol green (BCG) method and the Buret method. The globulin content was calculated as the difference between the total protein content and the albumin

content. In other words, the fibrinogen was calculated as included in the globulin for the sake of convenience.

The same procedure as described above was carried out separately without the addition of the purifying material by way of a blank test, to determine the albumin content and the globulin content. The amounts of albumin and globulin adsorbed in the experimental adsorption were calculated by subtracting the albumin content and the globulin content found in the experimental adsorption using the purifying material from the albumin content and the globulin content found in the blank test. The ratios of adsorption of albumin and globulin were calculated by dividing the values found in the experimental adsorption using the purifying material by the amounts of adsorption just mentioned. Further, the filtrate obtained in the procedure described above was tested for the IgG and IgM contents by the single radial immunodiffusion (SRID) method. The amounts of IgG and IgM adsorbed were found by subtracting the amounts found by this test from those found by the blank test and the ratios of adsorption of IgG and IgM were found by dividing the amounts mentioned above by the amounts of adsorption. The results were as shown in Table 8.

Controls 7 and 8

Purifying materials were prepared by following the procedure of Example 29, except that a solution of 0.1-M aspartic acid (Control 7) and a solution of 0.1-M phenylalanine (Control 8) each in 0.1-M carbonate buffer solution (pH 10) were used in the place of the solution of 0.05-M aspartylphenylalanine methyl ester in 0.1-M carbonate buffer solution (pH 10). These puryfing materials were put to use in the same experimental adsorption. The results were as shown in Table 8.

Control 9

An experimental adsorption was carried out by following the procedure of Example 19, except that chitosan beads (Lot

No. H-0132, produced by Fuji Spinning Co., Ltd. and marketed under trademark designation of "Chitopearl BCW3003") were used in the unmodified form as a purifying material. The results were as shown in Table 8.

Table 8

| | Carrier | Ligand | Adsorption capacity * | | | | | | | |
|---|---|---|---|---|---|---|---|---|---|---|
| | | | Amount of adsorption (mg/g) | | | | Adsorption rate (%) | | | |
| | | | Alb. | Glob. | Ig G | Ig M | Alb. | Glob. | Ig G | Ig M |
| Example 29 | Chitopearl BCW3003 | Aspartylphenyl-alanine methyl ester | 27.8 | 42.3 | 21.0 | 2.1 | 18.0 | 35.3 | 41.0 | 30.3 |
| Control 7 | Chitopearl BCW3003 | Aspartic acid | 29.1 | 35.9 | 18.0 | 1.5 | 18.9 | 30.0 | 36.4 | 21.0 |
| Control 8 | Chitopearl BCW3003 | Phenylalanine | 28.3 | 44.9 | 20.0 | 1.2 | 18.3 | 37.5 | 38.0 | 17.0 |
| Control 9 | Chitopearl BCW3003 | ——— | 26.2 | 40.7 | 18.0 | 1.0 | 17.0 | 34.0 | 36.0 | 14.0 |

* Alb and Glob stand for albumin and globulin respectively. The initial concentrations of proteins contained in the human plasma used for the experimental adsorption were 38.88 mg/ml of albumin, 29.92 mg/ml of globulin, 10.30 mg/ml of IgG, and 1.44 mg/ml of IgM.

It is clearly noted from the results of Table 8 that the purifying material conforming to the present invention (Example 29) produced by having aspartylphenylalanine methyl ester fixed as a ligand on the surface of chitosan beads as a carrier adsorbed IgG and IgM selectively as compared with the purifying materials using an amino acid as a ligand (Controls 7 and 8) and the purifying material using chitosan beads in the unmodified form (Control 9).

Example 30

A purifying material was prepared by following the procedure of Example 29, except that a dipeptide of glutamic acid and phenylalanine, i.e. α-L-glutamyl-L-phenylalanine, (produced by Kokusan Kagaku K.K.) was used in the place of aspartylphenylalanine methyl ester. This purifying material was put to use in the same experimental adsorption. The results were as shown in Table 9.

Example 31

A purifying material was prepared by following the procedure of Example 29, excepting a dipeptide of aspartic acid and tryptophan, i.e. L-tryptophyl-L-aspartic acid, (produced by Kokusan Kagaku K.K.) was used in the place of aspartylphenylalanine methyl ester. This puprifying material was put to use in the same experimental adsorption. The results were as shown in Table 9.

Controls 10 to 13

Purifying materials were prepared by following the procedure of Example 29, except that glutamic acid (Control 10), aspartic acid (Control 11), phenylalanine (Control 12), and tryptophan (Control 13) were used in the place of aspartylphenylalanine methyl ester. These purifying materials were put to use in the same experimental adsorption. The results were as shown in Table 9.

The experimental adsorption of the purifying materials of Example 29 and Control 7 to 9 and the experimental adsorption of the purifying materials of Examples 30 and 31 and Controls 10 to 13 were carried out on blood plasmas from

separate normal human s.

Table 9

| | Carrier | Ligand | Adsorption capacity * | | | | | | | |
|---|---|---|---|---|---|---|---|---|---|---|
| | | | Amount of adsorption (mg/g) | | | | Adsorption rate (%) | | | |
| | | | Alb. | Glob. | Ig G | Ig M | Alb. | Glob. | Ig G | Ig M |
| Example 30 | Chitopearl BCW3003 | α-L-glutamyl-L-phenylalanine | 26.8 | 43.5 | 14.0 | 1.8 | 18.3 | 33.6 | 50.4 | 22.6 |
| Example 31 | Chitopearl BCW3003 | L-triptphyl-L-aspartic acid | 26.6 | 41.7 | 23.6 | 1.5 | 18.2 | 32.2 | 49.4 | 18.6 |
| Control 10 | Chitopearl BCW3003 | glutamic acid | 28.9 | 33.6 | 22.0 | 1.4 | 19.8 | 26.4 | 46.0 | 17.0 |
| Control 11 | Chitopearl BCW3003 | Aspartic acid | 31.1 | 33.5 | 21.3 | 1.8 | 21.3 | 25.9 | 44.7 | 23.0 |
| Control 12 | Chitopearl BCW3003 | Phenylalanine | 26.8 | 41.9 | 22.2 | 1.5 | 18.4 | 32.3 | 46.4 | 18.6 |
| Control 13 | Chitopearl BCW3003 | Triyptophan | 24.9 | 42.9 | 21.8 | 1.1 | 17.1 | 33.2 | 45.5 | 13.2 |

* Alb and Glob stand for albumin and globulin respectively. The initial concentrations of proteins in the human plasma used in the experimental adsorption were 29.23 mg/ml of albumin, 25.90 mg/ml of globulin, 9.56 mg/ml of IgG, and 1.60 mg/ml of IgM.

It is clearly noted from the results of Table 9,similarly to the results of Table 8, that the purifying agents produced in accordance with this invention by having α-L-glutamyl-L-phenylalanine, a dipeptide, (Example 30) and L-tryptophyl-L-aspartic acid (Example 31) fixed on the surface of chitosan beads as a carrier adsorbed IgG and IgM with higher selectivity than the purifying materials using amino acids as a ligand (Controls 10 to 13).

[Industrial Applicability]

As described above, this invention is directed to a B lymphocyte separating material characterized by having fixed on the surface of a water-insoluble solid substance an organically synthesized ligand possessing high affinity for immunoglobulin. The B lymphocyte separating material, therefore, is capable of selectively sequestering and separating B lymphocytes. For example, it is capable of conveniently and quickly separating T lymphocytes and B lymphocytes in high yield with high purity from a lymphocyte fraction obtained from the peripheral blood or the vital tissue. Moreover, it effects this efficient separation while inflicting functional damage only sparingly on the separated T lymphocytes and B lymphocytes. It, therefore, can be utilized for the diagnosis and therapy of diseases associated with the immune function and for the basic technique of obtaining physiologically active substances originating in lymphocytes. The B lymphocyte separating material of this invention is capable of obtaining T lymphocytes and B lymphocytes with higher purity in higher yield more safely when the organically synthesized ligand is selected from the group consisting of heterocyclic compounds, peptides, and amino acids, more desirably the heterocyclic compound is selected from the group consisting of chemotherapeutics and derivatives thereof, pyrimidine-associated compounds, pyridine-associated compounds, and pyrazine-associated compounds, still more preferably the heterocyclic compound is selected from the group consisting

of sulfa drugs, compounds of sulfa drugs and coloring matters of pK 4.0 to 9.0, 2-thouracil, isonicotinic acid hydrazide, and luminol, preferably the peptide is an oligopeptide having a number of amino acids approximately in the range of 2 to 10, more preferably the oligopeptide is an oligopeptide originating in lysine and tyrosine or an aspartylphenylalanine alkyl ester, desirably the amino acid is one amino acid or a combination of two or more amino acids selected from the group consisting of valine, leucine, tyrosine, phenylalanine, isoleusine, trytophan, lysine, arginine, prolin, and aspartic acid and derivatives thereof, and the organically synthesized ligand is formed by the combination of arginine, aspartic acid, phenylalanine or proline with sulfathiazole. Further, the B lymphocyte separating material is capable of stably and efficiently separate B lymphocytes when the water-soluble solid substance is a particulate substance having an average particle diameter in the range of 0.05 to 5 mm and the organically synthesized ligand is fixed by a covalent bond to the surface of the water-insoluble solid substance.

Further, this invention is directed to a method for the separation of B lymphocytes, characterized by causing a B lymphocyte-containing liquid to contact a separating material having fixed on the surface of a water-insoluble solid substance an organically synthesized ligand possessing high affinity for immunoglobulin thereby allowing the separating material to sequester selectively B lymphocytes having immunoglobulin molecules (S-Ig) on the surface of membrane thereof. The method, therefore, attains convenient and highly accurate separation of B lymphocytes without requiring use of a concentrated blood serum or a physiologically active substance. Further, the method of this invention for the separation of B lymphocytes effects separation of T lymphocytes and B lymphocytes with higher purity in higher yield more safely than when it is used for the separation of a lymphocyte fraction, for example, when

the ligand fixed on the separating material is selected from the group consisting of heterocyclic compounds, peptides, and amino acids, more desirably the heterocyclic compound is selected from the group consisting of chemotherapeutics and derivatives thereof, pyrimidine-associated compounds pyridine-associated compounds, and pyrazine-associated compounds, still more preferably the heterocyclic compound is selected from the group consisting of sulfa drugs, compounds of sulfa drugs and coloring matters of pK 4.0 to 9.0, 2-thiouracil, isonicotinic acid hydrazide, and luminol, desirably the peptide is an oligopeptide having a number of amino acids approximately in the range of 2 to 10, more desirably the oligopeptide is an oligopeptide originating in lysine and tyrosine or an aspartylphenylalanine alkyl ester, desirably the amino acid is one amino acid or a combination of two or more amino acids selected from the group consisting of valine, leucine, tyrosine, phenylalanine, isoleusine, trytophan, lysine, arginine, prolin, and aspartic acid and derivatives thereof, and the organically synthesized ligand is formed by the combination of arginine, aspatic acid, phenylalanine or proline with sulfathiazole. The separation of T and B lymphocytes is attained more easily and more advantageously when an isotonic buffer solution is used as a washing liquid. When the separating material is treated with a bovine blood serum albumin and then is brought into contact with the lymphocyte-containing liquid, the method can be expected to bring about a still better effect because it is allowed to improve the yield in the operation of separation without impairing the purity of the separated T and B lymphocytes.

This invention is further directed to a B lymphocyte separating device characterized by being provided with a column packed with a separating material having fixed on the surface of a water-insoluble solid substance in a particular form an organically synthesized ligand possessing high affinity for immunoglobulin. This method, therefore, allows

the B lymphocyte-containing liquid such as, for example, a lymphocyte suspension to be efficiently brought into contact with the B lymphocyte separating material possessing highly satisfactory properties as described above and enables the separation of T lymphocytes and B lymphocytes to be successfully carried out quickly. This B lymphocyte separating device can be sterilized by a simple procedure and has only a remote possibility of inflicting damage to the cellular component in the B lymphocyte-containing liquid and, during the separation of a lymphocyte fraction, allows T lymphocytes and B lymphocytes to be separated with high puprity in high yield particularly when the column is made of polypropylene or polycarbonate, the column is provided between the outlet/inlet thereof and the separating material bed with a filter pervious to the cellular component in the B lymphocyte-containing liquid and impervious to the separating material, and this filter is made of polyester or polyamide.

Then, this invention is directed to a B lymphocyte separating material characterized by having fixed to the surface of a water-insoluble solid substance through the medium of a spacer an organically synthesized ligand possessing high affinity for immunoglobulin. This B lymphocyte separating material, therefore, is capable of selectively sequestering and separating B lymphocytes. From the lymphocyte fraction obtained from the peripheral blood or the vital tissue, for example, it is capable of easily and quickly separating T lymphocytes and B lymphocytes in very high yield with high purity. It inflicts functional damage only sparingly on the separated T lymphocytes and B lymphocytes. Thus, the B lymphocyte separating material can be utilized for the dianosis and therapy of diseases associated with immune function and for the basic technique of obtaining physiologically acitve substances originating in lymphocytes. The non-specific adsorption of T lymphocytes is further suppressed and the accuracy of

separation is further enhanced when the spacer in the B lymphocyte separating material of this invention has as its skeleton a hydrocarbon chain possessing at least two carbon atoms or a hydrocarbon chain containing a characteristic group and more desirably the spacer has as its skeleton a linear alkyl possessing a methylene repeating degree of not less than 4 or an ethylene oxide skeleton possessing a polymerization degree in the range of 1 to 50. Further, the B lymphocyte separating material of this invention is capable of obtaining T lymphocytes and B lymphocytes with high purity in high yield more safely when the organically synthesized ligand is selected from the group consisting of heterocyclic compounds, peptides, and amino acids, more desirably the heterocyclic compound is selected from the group consisting of chemotherapeutics and derivative thereof, pyrimidine-associated compounds, pyridine-associated compounds, and pyrazine-associated compounds, still more preferably the heterocyclic compound is selected from the group consisting of sulfa drugs, compounds of sulfa drugs and coloring matters of pK 4.0 to 9.0, 2-thiouracil, isonicotinic acid hydrazide, and luminol, desirably the peptide is an oligopeptide having a number of amino acids approximately in the range of 2 to 10, more preferably the oligopeptide is an oligopeptide originating in lysine and tyrosine or an aspartylphenylalanine alkyl ester, preferably the amino acid is one amino acid or a combination of two or more amino acids selected from the group consisting of valine, leucine, tyrosine, phenylalanine, isoleusine, trytophan, lysine, arginine, proline, and aspartic acid and derivatives thereof, and the organically synthesized ligand is formed by the combination of arginine, aspartic acid, phenylalanine or proline with sulfathiazole. It allows the separation of B lymphocytes to be attained more stably and more efficiently when the water-insoluble solid substance is a particulate substance having an average particle diameter in the range of 0.05 to 5 mm.

This invention is further directed to a method for the separation of B lymphocytes, characterized by causing a lymphocyte-containing liquid to contact a separating material having fixed on the surface of a water-insoluble solid substance through the medium of a space an organically synthesized ligand possessing high affinity for immunoglobulin thereby enabling the separating material to attain selective sequestration of B lymphocytes possessing immunoglobulin molecules (S-Ig) on the surface of the membrane thereof. This method, therefore, is capable of attaining convenient and highly accurate separation of B lymphocytes without requiring use of a concentrated blood serum or a physiologically active substance originating in an organism of different species during the operation of treatment. Moreover, this method can be expected to attain the separation of B lymphocytes with higher accuracy when the spacer in the separating material has as its background a hydrocarbon chain possessing at least two carbon atoms or a hydrocarbon chain containing a characteristic group and more desirably has an its skeleton a linear alkyl having a methylene repeating degree of not less than 4 or an ethylene oxide of a polymerization degree in the range of 1 to 50. Further, this method is enabled, during the separation of a lymphocyte fraction, for example, to accomplish the separation of T lymphocytes and B lymphocytes with higher purity in high yield more safely when the ligand fixed on the separating material is selected from the group consisting of heterocyclic compounds, peptides, and amino acids, more desirably the heterocyclic compound is selected from the group consisting of chemotherapeutics and derivatives thereof, pyrimidine-associated compounds, pyridine-associated compounds, and pyrazine-associated compounds, still more desirably the heterocyclic compound is selected from the group consisting of sulfa drugs, compounds of sulfa drugs and coloring matters of pK 4.0 to 9.0, 2-thiouracil, isonicotinic acid hydrazide, and luminol,

desirably the peptide is an oligopeptide having a number of amino acids approximately in the range of 2 to 10, more desirably the oligopeptide is an oligopeptide originating in lysine and tyrosine or an aspartylphenylalanine alkyl ester, desirably the amino acid is one amino acid or a combination of two or more amino acids selected from the group consisting of valine, leucine, tyrosine, phenylalanine, isoleusine, trytophan, lysine, arginine, proline, and aspartic acid and derivatives thereof, and the organically synthesized ligand is formed by the combination of arginine, aspartic acid, phenylalanine or proline with sulfathiazole. It is allowed to attain the separation of T and B lymphocytes easily and favorably and is expected to give highly desirable results when an isotonic buffer solution is used as the washing liquid. This invention is also directed to a B lymphocyte separating device characterized by being provided with a column packed with a separating material having fixed on the surface of a water-insoluble solid substance in a particulate form through the medium of a spacer an organically synthesized ligand possessing high affinity for immunoglobulin. This B lymphocyte separating device, therefore, is capable of establishing efficient contact between the B lymphocyte separating material possessing highly desirable properties as described above and the B lymphocyte-containing liquid such as, for example, a lymphocyte suspension and attaining the separation of B lymphocytes quickly and successfully. This B lymphocyte separating device can be sterilized by a simple procedure and has only a remote possibility of inflicting damage to the cellular component in the B lymphocyte-containing liquid and, during the separation of a lymphocyte fraction, allows T lymphocytes and B lymphocytes to be separated with high purity in high yield particularly when the column is made of polypropylene or polycarbonate, the column is provided between the outlet/inlet thereof and the separating material bed with a filter pervious to the cellular component in the

B lymphocyte-containing liquid and impervious to the separating material, and this filter is made of polyester or polyamide.

This invention, as described above, is directed further to a body fluid purifying material characterized by having a dipeptide or a derivative thereof fixed on the surface of a water-insoluble carrier. This body fluid purifying material, therefore, is capable of adsorbing and removing pathogenic substances, i.e. such proteins as immunoglobulin, immune complex, and immunity-associated soluble factors, efficiently with high selectivity and high safety and highly suitable as a purifying material for adsorbing and removing the pathogenic substances from the body fluid such as blood and consequently purifying the body fluid. It allows autoimmune diseases and immunity-associated diseases to be effectively treated by the method of automatic blood plasma purification. Not merely for the purpose of such therapy as mentioned above, the body fluid purifying material can be expected also to find extensive utility in the separation and purification of such specific components of the body fluid as immunoglobulin, immune complex, and immunity-associated soluble factors or in the test of the body fluid for such detrimental components. Since this body fluid purifying material possesses stable activity, it can be produced, sterilized, stored, transported, and maintained easily and is available at a low price. Further, the body fluid purifying material of this invention is allowed to manifest the aforementioned characteristic properties to better advantage and is expected to bring about a highly desirable effect when the dipeptide or a derivative thereof is a dipeptide of an acidic amino acid and an aromatic amino acid or a heterocyclic amino acid or a derivative thereof, desirably a dipeptide of aspartic acid or glutamic acid and phenylalanine, tyrosine, tryptophan, proline, or hydroxyproline or a derivative thereof, more desirably

aspartylphenylalanine or a derivative thereof, and most desirably aspartylphenylalanine methyl ester, the water-insoluble carrier is a particulate carrier preferably possessing an average particle diameter in the range of 0.05 to 5 mm, possessing a porous texture, and comprising a synthetic organic macromolecular substance, a natural organic macromolecular substance, or an inorganic substance, and particularly desirably porous acrylic ester type beads or porous chitosan beads, and the dipeptide or a derivative thereof is fixed by a covalent bond to the surface of the water-insoluble carrier or is fixed by a covalent bond through the medium of a spacer to the surface of the water-insoluble carrier.

This invention is further directed to a body fluid purifying device characterized by being provided with a column packed with a purifying material having a dipeptide or a derivative thereof fixed on the surface of a water-insoluble carrier. This body fluid purifying device, therefore, is capable of establishing easy and efficient contact between the body fluid purifying material possessing the highly desirable properties mentioned above and the body fluid, permitting the adsorption and removal of pathogenic substances from a patient's body to be carried out by the method of automatic blood plasma purification, and enabling the separation, purification, or examination of such pathogenic substances to be performed quickly and successfully. Even when the body fluid purifying device of this invention is subjected to a wet heat sterilizing treatment, it continues to exhibit a stable adsorbing activity without any decline of quality and provides safe treatment and operation. This body fluid purifying device can be sterilized by a simple procedure and has only a remote possibility of inflicting damage to the components in the body fluid being passed there through and permits the selective adsorption and removal of pathogenic substances to be attained more safely and more efficiently when the column

is made of polypropylene or polycarbonate and it is provided between the outlet/inlet thereof and the purifying material bed with a filter pervious to the body fluid components and impervious to the purifying material. This body fluid purifying device is enabled to manifest still better properties when the body fluid purifying material is obtained by having a dipeptide or a derivative thereof fixed on the surface of a water-soluble carrier in a particulate form, the dipeptide or a derivative thereof is a dipeptide of an acidic amino acid and an aromatic amino acid or a heterocyclic amino acid or a derivative thereof, desirably a dipeptide of aspartic acid or glutamic acid and phenylalanine, tyrosine, tryptophan, proline, or hydroxyproline or a derivative thereof, more desirably aspartylphenylalanine or a derivative thereof, and most desirably aspartylphenylalanine methyl ester, the water-insoluble carrier is a particulate carrier preferably possessing an average particle diameter in the range of 0.05 to 5 mm, possessing a porous texture, and comprising a synthetic organic macromolecular substance, a natural organic macromolecular substance, or an inorganic substance, and particularly desirably porous acrylic ester type beads or porous chitosan beads, and the dipeptide or a derivative thereof is fixed by a covalent bond to the surface of the water-insoluble carrier or is fixed by a covalent bond through the medium of a spacer to the surface of the water-insoluble carrier.

In addition, this invention is directed to a method for automatic blood plasma purification, which comprises separating blood led out of a patient into a blood cell component and a blood plasma component, causing the blood plasma component so obtained to contact body fluid purifying material having a dipeptide or a derivative thereof fixed on the surface of a water-insoluble carrier, mixing the blood plasma component resulting from the treatment with the body fluid purifying material with the blood cell component, and

returning the resultant mixture to the patient. This method, therefore, is capable of selectively and efficiently removing pathogenic substances from the blood plasma component without removing albumin and other useful components prevent in the blood plasma and enabling the autoimmuno diseases and immunity-associated diseases mentioned above to be effectively cured and consequently contributing immensely to the field of therapy.

The method of this invention for automatic blood plasma purification can be expected to produce more conspicuous therapeutic effect when the dipeptide or a derivative is a dipeptide of an acidic amino acid and an aromatic amino acid or a heterocyclic amino acid or a derivative thereof, desirably a dipeptide of aspartic acid or glutamic acid and phenylalanine, tyrosine, tryptophan, proline, or hydroxyproline or a derivative thereof, more desirably aspartylphenylalanine or a derivative thereof, and most desirably aspartylphenylalanine methyl ester, the water-insoluble carrier is a particulate carrier preferably possessing an average particle diameter in the range of 0.05 to 5 mm, possessing a porous texture, and comprising a synthetic organic macromolecular substance, a natural organic macromolecular substance, or an inorganic substance, and particularly desirably porous acrylic ester type beads or porous chitosan beads, and the dipeptide or a derivative thereof is fixed by a covalent bond to the surface of the water-insoluble carrier or is fixed by covalent bond through the medium of a spacer to the surface of the water-insoluble carrier.

WHAT IS CLAIMED IS:

1. A B lymphocyte separating material, characterized by the fact that an organically synthesized ligand possessing affinity for immunoglobulin is fixed on the surface of a water-insoluble solid substance.

2. A B lymphocyte separating material according to claim 1, wherein said organically synthesized ligand is selected from the group consisting of heterocyclic compounds, peptides, and amino acids.

3. A B lymphocyte separating material according to claim 2, wherein said heterocyclic compound is selected from the group consisting of chemotherapeutics and derivatives thereof, pyrimidine-associated compounds, pyridine-associated compounds, and pyrazine-associated compounds.

4. A B lymphocyte separating material according to claim 3, wherein said heterocyclic compound is selected from the group consisting of sulfa drugs, compounds formed of sulfa drugs with coloring matters of pK 4.0 to 9.0, 2-thiouracil, isonicotinic acid hydrazide, and luminol.

5. A B lymphocyte separating material according to claim 2, wherein said peptide is an oligopeptide having the number of amino acids approximately in the range of 2 to 10.

6. A B lymphocyte separating material according to claim 5, wherein said oligopeptide is made of lysine, and tyrosine, or an aspartylphenylalanine alkyl ester.

7. A B lymphocyte separating material according to claim 2, wherein said amino acid is one amino acid or a combination of two or more amino acids selected from the group consisting of valine, leucine, tyrosine, phenylalanine, isoleucine, tryptophan, lysine, arginine, proline, and aspartic acid, and derivatives thereof.

8. A B lymphocyte separating material according to claim 1 or claim 2, wherein said organically synthesized ligand is selected formed by the combination of arginine, aspartic acid, phenylalanine, and proline with sulfathiazole.

9. A B lymphocyte separating material according to any of claims 1 to 8, wherein said water-insoluble solid substance comprises a synthetic organic macromolecular substance, a natural organic macromolecular substance, or an inorganic substance.

10. A B lymphocyte separating material according to any of claims 1 to 9, wherein said water-insoluble solid substance comprises particles possessing an average particle diameter in the range of 0.05 to 5 mm.

11. A B lymphocyte separating material according to any of claims 1 to 10, wherein said organically synthesized ligand is fixed by a covalent bond on the surface of the water-insoluble solid substance.

12. A method for the separation of B lymphocytes, characterized by the fact that a B lymphocyte-containing liquid is caused to contact a separating material obtained by fixing on the surface of a water-insoluble solid substance an organically synthesized ligand possessing affinity for immunoglobulin so as to effect selective sequestration of B lymphocytes possessing an immunoglobulin molecule (S-Ig) on said membrane surface.

13. A method for the separation of B lymphocytes according to claim 12, wherein ligand fixed on said separating material is selected from the group consisting of heterocyclic compounds, peptides, and amino acids.

14. A method for the separation of B lymphocytes according to claim 13, wherein said heterocyclic compound is selected from the group consisting of chemotherapeutics and derivatives thereof, pyrimidine-associated compounds, pyridine-associated compounds, and pyrazine-associated compounds.

15. A method for the separation of B lymphocytes according to claim 14, wherein said heterocyclic compound is selected from the group consisting of sulfa drugs, compounds formed of sulfa drugs with coloring matters of pK 4.0 to 9.0, 2-thiouracil, isonicotinic acid hydrazide, and luminol.

16. A method for the separation of B lymphocytes according to claim 13, wherein said peptide is an oligopeptide having the number of amino acids approximately in the range of 2 to 10.

17. A method for the separation of B lymphocytes according to claim 16, wherein said oligopeptide is made of lysine, and tyrosine, or an aspartylphenylalanine alkyl ester.

18. A method for the separation of B lymphocytes according to claim 13, wherein said amino acid is one amino acid or a combination of two or more amino acids selected from the group consisting of valine, leucine, tyrosine, phenylalanine, isoleucine, tryptophan, lysine, arginine, proline, and aspartic acid, and derivatives thereof.

19. A method for the separation of B lymphocytes according to claim 12, wherein said ligand fixed on said separating material is the combination of arginine, aspartic acid, phenylalanine, or proline with sulfathiazole.

20. A method for the separation of B lymphocytes according to any of claims 12 to 19, wherein said water-insoluble solid substance comprises a synthetic organic macromolecular substance, a natural organic macromolecular substance, or an inorganic substance.

21. A method for the separation of B lymphocytes according to claim 12, wherein said an isotonic buffer solution is used as said purifying liquid.

22. A method for the separation of B lymphocytes according to claim 12, wherein said separating material is treated with bovine serum albumin prior to said contact thereof with said lymphocyte-containing liquid.

23. A B lymphocyte separating device, characterized by comprising a column packed with a separating material obtained by fixing on the surface of a water-insoluble solid substance an organically synthesixed ligand possessing affinity for immune globulin.

24. A B lymphocyte separating device according to claim 23, wherein said column is made of polypropylene or polycarbonate.

25. A B lymphocyte separating device according to claim 23 or claim 24, wherein said column is provided between the inlet/outlet thereof and the separating material bed thereof with a filter possessing meshes pervious to the cell component of said B lymphocyte-containing liquid and impervious to the separating material.

26. A B lymphocyte separating device according to claim 25, wherein said filter is made of polyester or polyamide.

27. A B lymphocyte separating material, characterized by the fact that an organically synthesized ligand possessing affinity for immunoglobulin is fixed on the surface of a water-insoluble solid substance through the medium of a spacer.

28. A B lymphocyte separating material according to claim 27, wherein said spacer has at the skeleton thereof a hydrocarbon chain possessing at least two carbon atoms or a hydrocarbon chain containing a characteristic group.

29. A B lymphocyte separating material according to claim 28, wherein the repetition of methylene in said spacer has as the skeleton thereof at least four linear alkyls.

30. A B lymphocyte separating material according to claim 28, wherein said spacer possesses an ethylene oxide skeleton having a polymerization degree in the range of 1 to 50.

31. A B lymphocyte separating material according to any of claims 27 to 30, wherein said organically synthesized ligand is selected from the group consisting of heterocyclic compounds, peptides, and amino acids.

32. A B lymphocyte separating material according to claim 31, wherein said heterocyclic compound is selected from the group consisting of chemotherapeutics and derivatives thereof, pyrimidine-associated compounds,

pyridine-associated compounds, and pyrazine-associated compounds.

33. A B lymphocyte separating material according to claim 32, wherein said heterocyclic compound is selected from the group consisting of sulfa drugs, compounds formed of sulfa drugs with coloring matters of pK 4.0 to 9.0, 2-thiouracil, isonicotinic acid hydrazide, and luminol.

34. A B lymphocyte separating material according to claim 33, wherein said peptide is an oligopeptide having the number of amino acids approximately in the range of 2 to 10.

35. A B lymphocyte separating material according to claim 34, wherein said oligopeptide is made of lysine, and tyrosine, or an aspartylphenylalanine alkyl ester.

36. A B lymphocyte separating material according to claim 31, wherein said amino acid is one among acid or a combination of two or more amino acids selected from the group consisting of valine, leucine, tyrosine, phenylalanine, isoleucine, tryptophan, lysine, artinine, proline, and aspartic acid, and derivatives thereof.

37. A B lymphocyte separating material according to claim 31, wherein said organically synthesized ligand is selected formed by the combination of arginine, aspartic acid, phenylalanine, and proline with sulfathiazole.

38. A B lymphocyte separating material according to any of claims 27 to 37, wherein said water-insoluble solid substance comprises a synthetic organic macromolecular substance, a natural organic macromolecular substance, or an inorganic substance in a particulate form.

39. A B lymphocyte separating material according to any of claims 27 to 38, wherein said water-insoluble solid substance comprises particles possessing an average particle diameter in the range of 0.05 to 5 mm.

40. A method for the separation of B lymphocytes, characterized by comprising a column packed with a separating material obtained by fixing on the surface of a water-insoluble solid substance through the medium of a

spacer an organically synthesized ligand possessing affinity for immunoglobulin.

41. A method for the separation of B lymphocytes according to claim 40, wherein said spacer has as the skeleton thereof a hydrocarbon chain possessing at least two carbon atoms or a hydrocarbon chain containing a characteristic group.

42. A method for the separation of B lymphocytes according to claim 41, wherein the repetition of methylene in said spacer has as the skeleton thereof at least four linear alkyls.

43. A method for the separation of B lymphocytes according to claim 41, wherein said spacer possesses an ethylene oxide skeleton having a polymerization degree in the range of 1 to 50.

44. A method for the separation of B lymphocytes according to any of claims 40 to 43, wherein said ligand fixed on said separating material is selected from the group consisting of heterocyclic compounds, peptides, and amino acids.

45. A method for the separation of B lymphocytes according to claim 44, wherein said heterocyclic compound is selected from the group consisting of chemotherapeutics and derivative thereof, pyrimidine-associated compounds, pyridine-associated compounds, and pyrazine-associated compounds.

46. A method for the separation of B lymphocytes according to claim 45, wherein said heterocyclic compound is selected from the group consisting of sulfa drugs, compounds formed of sulfa drugs with coloring matters of pK 4.0 to 9.0, 2-thiouracil, isonicotinic acid hydrazide, and luminol.

47. A method for the separation of B lymphocytes according to claim 44, wherein said peptide is an oligopeptide having the number of amino acids approximately in the range of 2 to 10.

48. A method for the separation of B lymphocytes according to claim 47, wherein said oligopeptide is made of lysine, and tyrosine, or an aspartylphenylalanine alkyl ester.

49. A method for the separation of B lymphocytes according to claim 44, wherein said amino acid is one amino acid or a combination of two or more amino acids selected from the group consisting of valine, leucine, tyrosine, phenylalanine, isoleucine, tryptophan, lysine, arginine, proline,and aspartic acid, and derivatives thereof.

50. A method for the separation of B lymphocytes according to claim 44, wherein said ligand fixed on said separating material is selected from the group consisting of heterocyclic compounds, peptides, and amino acids.

51. A method for the separation of B lymphocytes according to any of claims 40 to 50, wherein said water-insoluble solid substance comprises a synthetic organic macromolecular substance, a natural organic macromolecular substance, or an inorganic substance.

52. A method for the separation of B lymphocytes according to claim 40, wherein an isotonic buffer solution is used as a purifying liquid.

53. A B lymphocyte separating device, characterized by comprising a column packed with a separating material obtained by fixing on the surface of a water-insoluble solid substance in a particulate form through the medium of a spacer an organically synthesized ligand possessing affinity for immunoglobulin.

54. A B lymphocyte separating device according to claim 53, wherein said column is made of polypropylene or polycarbonate.

55. A B lymphocyte separating device according to claim 53 or claim 54, wherein said column is provided between the inlet/outlet thereof and the separating material bed thereof with a filter possessing meshes pervious to the cell

component of said B lymphocyte-containing liquid and impervious to the separating material.

56. A B lymphocyte separating device according to claim 55, wherein said filter is made of polyester or polyamide.

57. A body fluid purifying material, characterized by having a dipeptide or a derivative thereof fixed on the surface of a water-insoluble carrier.

58. A body fluid purifying material according to claim 57, wherein said dipeptide or the derivative thereof is a dipeptide of an acidic amino acid and an aromatic amino acid or a heterocyclic amino acid.

59. A body fluid purifying material according to claim 57 or claim 58, wherein said dipeptide or the derivative thereof is a dipeptide of aspartic acid or glutamic acid with phenylalanine, tyrosine, tryptophan, proline, or hydroxyproline or a derivative thereof.

60. A body fluid purifying material according to any of claims 57 to 59, wherein said dipeptide or the derivative thereof is aspartylphenylalanine or a derivative thereof.

61. A body fluid purifying material according to claim 60, wherein said dipeptide or the derivative thereof is an aspartylphenylalanine methyl ester.

62. A body fluid purifying material according to any of claims 57 to 61, wherein said water-insoluble carrier is a particulate carrier.

63. A body fluid purifying material according to claim 62, wherein said particulate carrier possesses an average particle diameter in the range of 0.05 to 5 mm.

64. A body fluid purifying material according to any of claims 57 to 63, wherein said water-insoluble carrier is a porous substance.

65. A body fluid purifying material according to any of claims 57 to 64, wherein said water-insoluble carrier is made of a synthetic organic macromolecular substance, a natural organic macromolecular substance, or an inorganic substance.

66. A body fluid purifying material according to any of claims 57 to 65, wherein said water-insoluble carrier comprises porous acrylic ester type particles or porous chitosan particles.

67. A body fluid purifying material according to any of claims 57 to 66, wherein said dipeptide or the derivative thereof is fixed by a covalent bond to the surface of the water-insoluble carrier.

68. A body fluid purifying material according to any of claims 57 to 66, wherein said dipeptide or the derivative thereof is fixed by a covalent bond through the medium of a spacer to the surface of the water-insoluble carrier.

69. A body fluid purifying device, characterized by comprising a column packed with a purifying material having a dipeptide or a derivative thereof fixed on the surface of a water-insoluble carrier.

70. A body fluid purifying device according to claim 69, which has undergone a wet-hot sterilizing treatment.

71. A body fluid purifying device according to claim 69 or claim 70, wherein said purifying material has a dipeptide or a derivative thereof fixed on the surface of the water-insoluble carrier.

72. A body fluid purifying device according to any of claims 69 to 71, wherein said column is made of polypropylene or polycarbonate.

73. A body fluid purifying device according to any of claims 69 to 72, wherein said column is provided between the inlet/outlet thereof and the purifying material bed with a filter possessing meshes pervious to the body fluid component and impervious to the purifying material.

74. A body fluid purifying device according to any of claims 69 to 73, wherein said dipeptide or the derivative thereof is a dipeptide of an acidic amino acid with an aromatic amino acid or a heterocyclic amino acid or a derivative thereof.

75. A body fluid purifying device according to any of claims 69 to 74, wherein said dipeptide or the derivative thereof is a dipeptide of aspartic acid or glutamic acid with phenylalanine, tyrosine, tryptophan, proline, or hydroxyproline or a derivative thereof.

76. A body fluid purifying device according to any of claims 69 to 75, wherein dipeptide or the derivative thereof is aspartylphenylalanine or a derivative thereof.

77. A body fluid purifying device according to claim 76, wherein dipeptide or the derivative thereof is an aspartylphenylalanine methyl ester.

78. A body fluid purifying device according to any of claims 69 to 77, wherein said particulate carrier possesses an average particle diameter in the range of 0.05 to 5 mm.

79. A body fluid purifying device according to any of claims 69 to 78, wherein said water-insoluble carrier is a porous substance.

80. A body fluid purifying device according to any of claims 69 to 79, wherein said water-insoluble carrier is made of a synthetic organic macromolecular substance, a natural organic macromolecular substance, or an inorganic substance.

81. A body fluid purifying device according to any of claims 69 to 80, wherein said water-insoluble carrier comprises porous acrylic ester type particles of porous chitosan particles.

82. A body fluid purifying device according to any of claims 69 to 81, wherein said dipeptide or the derivative thereof is fixed by a covalent bond to the surface of the water-insoluble carrier.

83. A body fluid purifying device according to any of claims 69 to 82, wherein said dipeptide or the derivative thereof is fixed by a covalent bond through the medium of a spacer to the surface of the water-insoluble carrier.

84. A method for automatic blood plasma purification, comprising the steps of separating the blood led out of a

patient into a blood component and a blood plasma component, causing the blood plasma component to contact a body fluid purifying material set forth in claim 57, causing the blood plasma component resulting from the treatment with the body fluid purifying material to be mixed with the blood component, and returning the resultant mixture to the patient.

85. A method for automatic blood plasma purification, comprising the steps of separating the blood led out of a patient into a blood component and a blood plasma component, causing the blood plasma component to contact a body fluid purifying material set forth in claim 68, causing the blood plasma component resulting from the treatment with the body fluid purifying material to be mixed with the blood component, and returning the resultant mixture to the patient.

# FIG.1

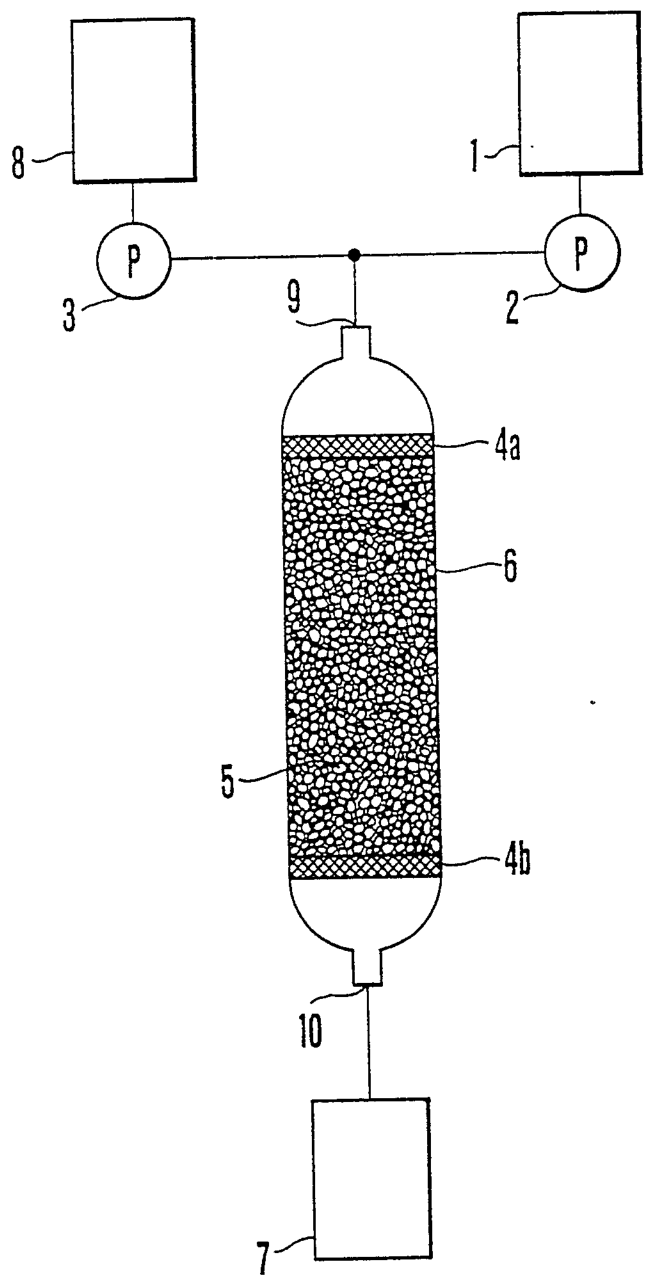

# F I G.2

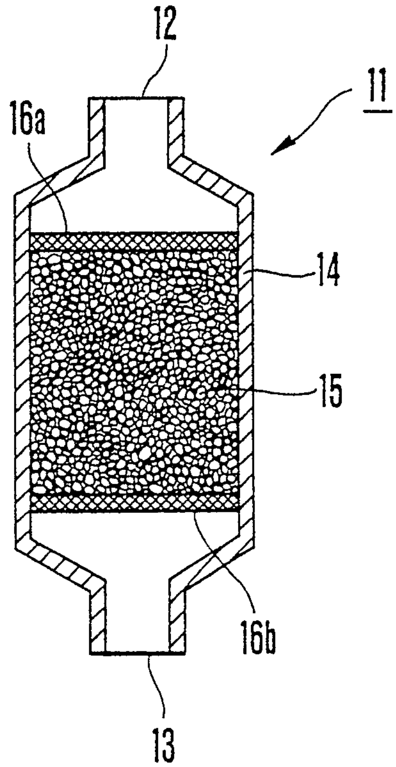


# F I G.3

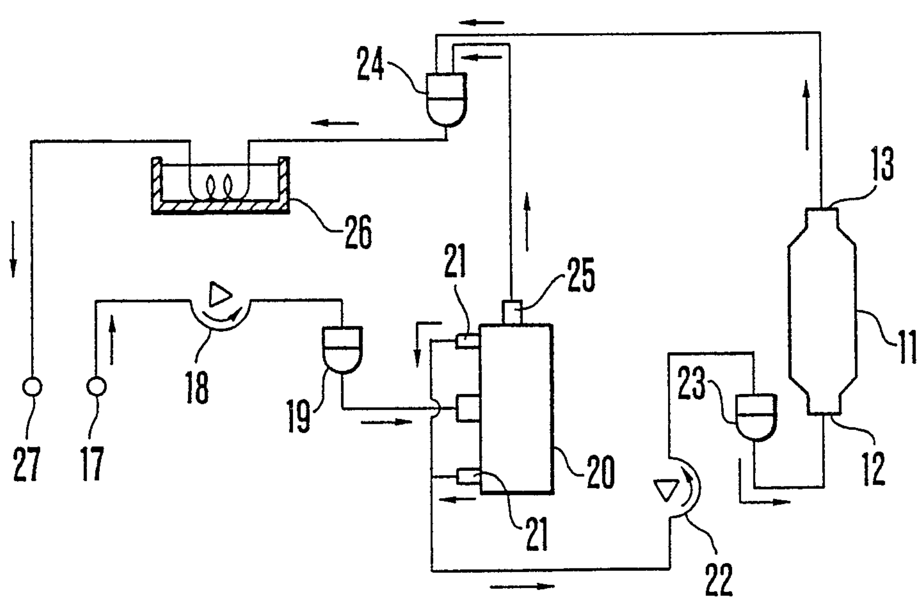

# INTERNATIONAL SEARCH REPORT

International Application No PCT/JP88/00359

**I. CLASSIFICATION OF SUBJECT MATTER** (if several classification symbols apply, indicate all)

According to International Patent Classification (IPC) or to both National Classification and IPC

Int.Cl$^4$ B01J20/22-20/26, A61M1/36, A61K35/14, G01N33/50

**II. FIELDS SEARCHED**

Minimum Documentation Searched

| Classification System | Classification Symbols |
|---|---|
| IPC | B01J20/22-20/26, A61M1/36, A61K35/14, G01N33/50 |

Documentation Searched other than Minimum Documentation to the Extent that such Documents are Included in the Fields Searched

**III. DOCUMENTS CONSIDERED TO BE RELEVANT**

| Category * | Citation of Document, with indication, where appropriate, of the relevant passages | Relevant to Claim No. |
|---|---|---|
| Y | JP, B2, 60-3367 (Asahi Chemical Industry Co., Ltd.) 28 January 1985 (28. 01. 85) Page 1 (Family: none) | 1, 2, 12, 13, 23, 24, 53, 55 |
| P | JP, A, 63-9450 (Terumo Corporation) 16 January 1988 (16. 01. 88) Page 1 (Family: none) | 1-4, 12-15, 23-33, 53-56 |
| X | JP, B2, 59-36961 (Asahi Chemical Industry Co., Ltd.) 6 September 1984 (06. 09. 84) Page 1 (Family: none) | 1, 2, 12, 13, 23, 24, 53, 55 |
| X | JP, A, 58-170717 (Turuta Teiji) 7 October 1983 (07. 10. 83) Page 1 (Family: none) | 1, 2, 12, 13, 23, 24, 53, 55 |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 21, 1988 (21. 06. 88) | July 4, 1988 (04. 07. 88) |
| International Searching Authority | Signature of Authorized Officer |
| Japanese Patent Office | |